(19) Europäisches Patentamt
European Patent Office
Office européen des brevets

(11) Numéro de publication : **0 366 542 B1**

# (12) FASCICULE DE BREVET EUROPEEN

(45) Date de publication du fascicule du brevet : **14.07.93 Bulletin 93/28**

(51) Int. Cl.$^5$ : **C07C 233/43,** A61K 7/13, C07C 233/44, C07C 271/28

(21) Numéro de dépôt : **89402940.4**

(22) Date de dépôt : **25.10.89**

## (54) Ortho-aminophénols 5-substitués procédé pour les préparer et leur utilisation pour la teinture d'oxydation des fibres kératiniques.

(30) Priorité : **28.10.88 FR 8814204**

(43) Date de publication de la demande : **02.05.90 Bulletin 90/18**

(45) Mention de la délivrance du brevet : **14.07.93 Bulletin 93/28**

(84) Etats contractants désignés : **AT BE CH DE ES FR GB GR IT LI NL SE**

(56) Documents cités : **DE-A- 3 641 825**

(73) Titulaire : **L'OREAL**
**14, Rue Royale**
**F-75008 Paris (FR)**

(72) Inventeur : **Junino, Alex**
**16, rue Docteur Bergonié**
**F-93180 Livry-Gargan (FR)**
Inventeur : **Andrean, Hervé**
**203, rue d'Alésia**
**F-75014 Paris (FR)**
Inventeur : **Lang, Gérard**
**44, avenue Lacour**
**F-95210 Saint-Gratien (FR)**

(74) Mandataire : **Casalonga, Axel et al**
**BUREAU D.A. CASALONGA - JOSSE**
**Morassistrasse 8**
**W-8000 München 5 (DE)**

EP 0 366 542 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet des ortho-amino phénols 5-substitués, leur procédé de préparation et leur utilisation dans des compositions tinctoriales pour la teinture des fibres kératiniques et en particulier des cheveux humains.

Ces compositions tinctoriales sont utilisées jour la coloration dite coloration d'oxydation ou teinture permanente. Ce procédé de coloration permet de teindre les fibres kératiniques, notamment les cheveux blancs, dans un grand nombre de nuances.

La teinture des fibres kératiniques par la coloration dite d'oxydation est connue. Cette teinture utilise des précurseurs de colorants d'oxydation appelés également base d'oxydation qui sont incolores, mais au contact d'un oxydant, développent dans les fibres kératiniques une coloration durable. On connaît comme précurseurs de colorants d'oxydation les paraphénylène diamines, les orthophénylènediamines, les para-amino phénols, les ortho-aminophénols, substitués ou non substitués. Ces précurseurs de colorants d'oxydation appelés ci-après "précurseurs" peuvent être mélangés avec un ou plusieurs composés appelés "coupleurs". Ces coupleurs généralement choisis parmi les métadiamines, les méta-aminophénols, les métadiphénols et les phénols permettent de modifier les nuances qui seraient obtenues avec le ou les précurseurs.

Le brevet français 2.460.664 et le brevet US 4.370.142 décrivent des compositions tinctoriales et un procédé de teinture des cheveux à base de para-phénylènediamine et d'ortho-aminophénol, dans des rapports très particuliers et en l'absence de coupleurs, pour obtenir des couleurs allant du châtain au noir.

DE-A-3.641.825 enseigne un procédé de préparation de 2-amino 5-acylaminophénols utilisés comme intermédiaires pour la synthèse de coupleurs pour l'obtention de couleurs bleu-vert en photographie. Cependant, cette référence n'enseigne pas qu'un choix spécifique des deux substituants variables de la formule générale permette d'obtenir des composés utilisables comme précurseurs ou comme coupleurs pour la teinture des fibres kératiniques et en particulier de cheveux humains.

Le problème que résout la présente invention est de choisir parmi les 2-amino 5-acylaminophénols divulgués comme intermédiaires par DE-A-3.641.825, ceux utilisables pour la teinture des fibres kératiniques et en particulier des cheveux humains.

On a découvert que les ortho-aminophénols substitués en position 5 seuls ou associés soit à d'autres précurseurs, soit à des coupleurs, soit aux deux permettent de teindre les fibres kératiniques et en particulier les cheveux en un riche éventail de nuances qui n'étaient pas obtenues avec les compositions décrites dans les brevets susindiqués.

La présente invention a pour objet les nouveaux composés que sont les ortho-aminophénols 5-substitués répondant à la formule générale (I) ci-après, leurs sels d'addition avec un acide et leurs phénates.

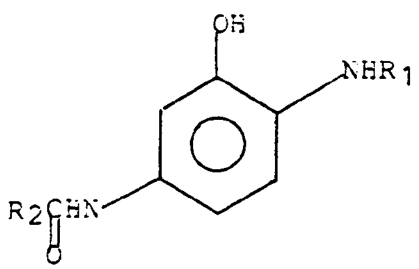

(I)

Dans cette formule :

$R_1$ représente un atome d'hydrogène, un radical alkyle ayant 1 à 4 atomes de carbone, un radical hydroxyalkyle ayant 1 à 4 atomes de carbone;

$R_2$ représente, indépendamment de $R_1$, un radical alkyle ayant 1 à 4 atomes de carbone, un radical alcoxy ayant 1 à 4 atomes de carbone, un radical benzyle.

Un autre objet de l'invention est l'utilisation des composés de formule (I) ou de leurs sels d'addition avec un acide ou de leurs phénates, soit seul, soit en association avec des coupleurs. Dans ce cas, les composés de formule (I) jouent, en teinture d'oxydation, le rôle de précurseurs de colorants d'oxydation. En association avec d'autres précurseurs les composés de formule générale (I) ou leurs sels ou leurs phénates jouent le rôle de coupleurs pour la teinture des fibres kératiniques et en particulier de cheveux humains.

La présente invention a également pour objet une composition tinctoriale pour fibres kératiniques et en particulier pour cheveux humains, comprenant dans un véhicule aqueux, un ou plusieurs composés de formule (I).

Les compositions tinctoriales contenant un ou plusieurs composés de formule (I), soit seuls, soit en association avec un précurseur ou un coupleur, permettant de conférer aux cheveux une grande variété de nuances. Variétés de nuances allant des bleus aux pourpres lorsque le composé (I) est associé à un précurseur choisi parmi les p-phénylènediamines, des rouges aux orangés lorsqu'il est associé à un précurseur choisi parmi les p-aminophénols. Utilisé seul ou en association avec un "coupleur", le composé (I) confère aux cheveux des nuances allant des blonds et des beiges aux orangés.

Ainsi, les composés de formule (I) offrent au chimiste cosmétologue un riche éventail de nuances permettant une grande richesse de formulation.

L'invention vise également le procédé de préparation des composés de formule (I) :

Les composés de formule (I) sont obtenus par un procédé en quatre étapes, selon le schéma réactionnel ci-après, dans lequel $R_1$ et $R_2$ ont la signification ci-dessus définie, et $B_z$ désigne benzyle.

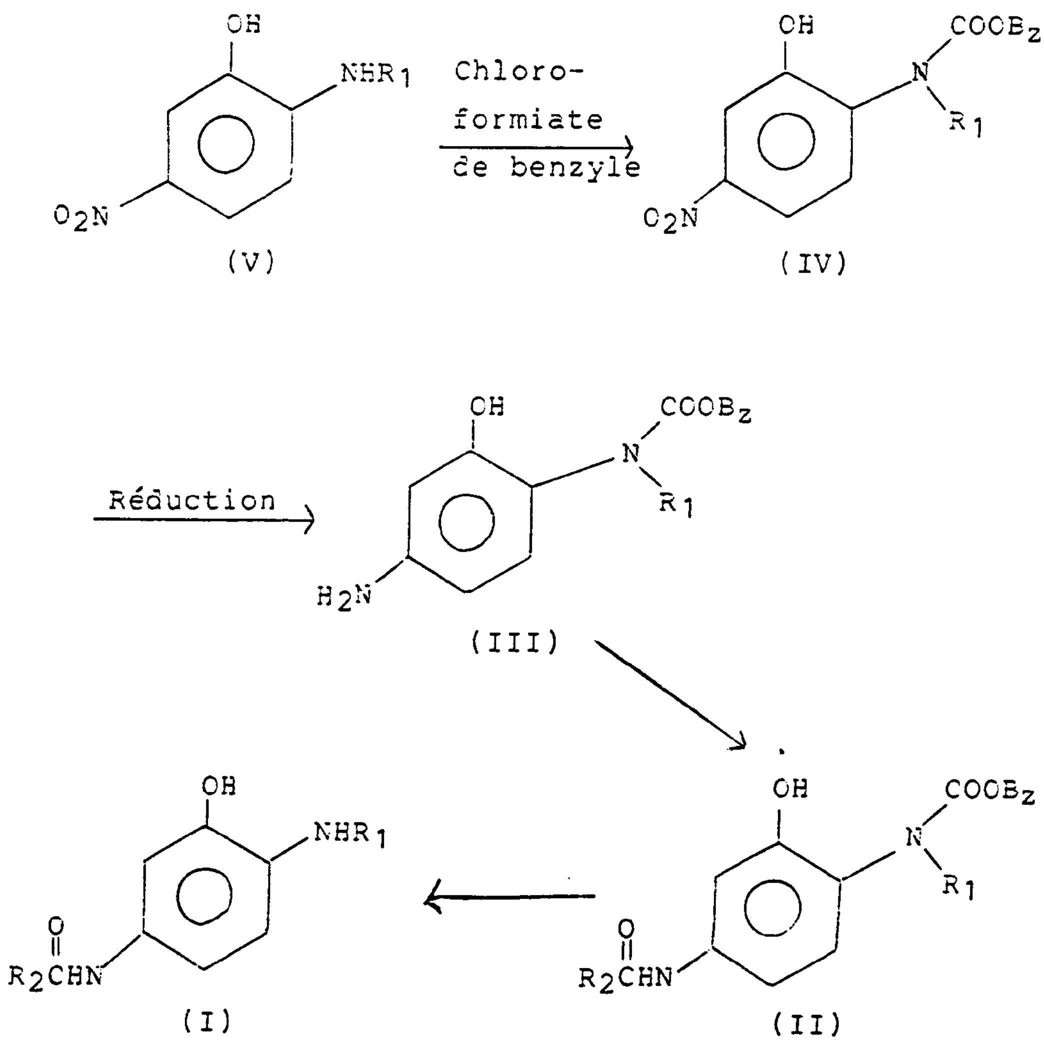


(V) (IV) (III) (II) (I)

1ère étape :

La condensation du chloroformiate de benzyle sur l'aminonitrophénol de formule (V) s'effectue dans un solvant polaire, tel que le dioxane, le diméthylformamide, le diméthylsulfoxyde ou la N-méthylpyrrolidone, à une température ne dépassant pas 100°C, en présence d'un capteur d'acide chlorhydrique qui est de préférence le carbonate de calcium.

2ème étape :

La réduction du nitrophénol de formule (IV), doit s'effectuer sans provoquer le clivage du carbamate de

benzyle. Cette réduction peut s'effectuer :

(i) par hydrogénation catalytique en utilisant comme catalyseur le nickel de Raney en présence de solvants usuels tels que l'acétate d'éthyle ou un alcool en $C_1$-$C_4$ et de préférence l'éthanol, sous une pression d'hydrogène inférieure ou égale à $10^6$ Pascal (10 bar);
(ii) réduction par transfert d'hydrogène, en utilisant comme catalyseur le nickel de Raney, comme donneur d'hydrogène l'hydrazine et comme solvant un mélange hydroalcoolique;
(iii) réduction par l'hydrosulfite de sodium, en utilisant comme solvant l'eau;
(iv) réduction par le fer acétique dans un solvant, tel que l'eau ou l'éthanol.
Ces réactions de réduction s'effectuent à une température comprise entre 20 et 100°C.

3ème étape :

L'acylation du composé (III) se réalise au moyen d'un chlorure d'acide ou d'un alkylchloroformiate ou encore lorsque $R_2$ représente un radical alkyle, acylation de la fonction amino par un anhydride d'acide.

4ème étape :

La coupure du radical benzyloxy, s'effectue soit par hydrogénation catalytique en utilisant comme catalyseur le palladium, soit par transfert d'hydrogène en utilisant comme donneur d'hydrogène le cyclohexène en présence de palladium.

Ces réactions s'effectuent dans un solvant alcoolique ou hydroalcoolique, en utilisant un alcool en $C_1$-$C_4$.

Ces dernières réactions sont décrites dans "Protective groups in organic synthesis"par . W. GREEN, John Wiley éditeur, p. 239 (1981).

L'invention est illustrée par les exemples de préparation non limitatifs ci-après.

EXEMPLE DE PREPARATION 1

Préparation du chlorhydrate de 5-acétamido 2-amino phénol.

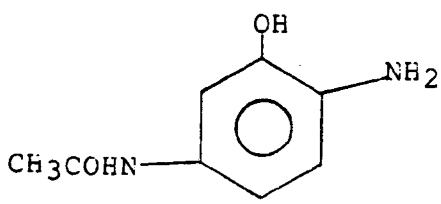

, HCl

1ère étape :

Préparation du 2-(N-benzyloxycarbonyl)amino 5-nitrophénol.

A 3,25 moles (500 g) de 2-amino 5-nitro phénol et 204,5 g de carbonate de calcium dans 1,5 litre de dioxane portés à 70°C, on ajoute en 45 minutes 3,58 moles (536 ml) de chloroformiate de benzyle (95%) en maintenant la température entre 70°C et 80°C. L'addition terminée, on maintient le chauffage 30 minutes supplémentaires. Les sels minéraux présents dans le milieu réactionnel sont éliminés par filtration à chaud. Après addition d'eau glacée au filtrat, le produit attendu cristallise. Après filtration et séchage, le produit obtenu est recristallisé de l'acide acétique. Il fond à 200°C.

L'analyse du produit obtenu donne les résultats suivants :

| Analyse | Calculé pour $C_{14}H_{12}N_2O_5$ | Trouvé |
|---|---|---|
| C | 58,33 | 58,38 |
| H | 4,20 | 4,17 |
| N | 9,72 | 9,69 |
| O | 27,75 | 27,60 |

2ème étape :

Préparation du 5-amino 2-(N-benzyloxycarbonyl) aminophénol.

Dans un autoclave, on soumet à une pression de $8x10^5$ Pascal (8 bars) d'hydrogène 0,53 mole (153 g) de 2-(benzyloxycarbonyl)amino 5-nitrophénol dans 1 litre d'éthanol en présence de 60 g de nickel de Raney (50% humide). Après 1 heure à 65°C, le milieu réactionnel est filtré chaud afin d'éliminer le catalyseur. Par refroidissement du filtrat, le produit attendu cristallise. Après essorage et séchage, le produit obtenu est recristallisé de l'éthanol à 96°. Il fond à 148°C.

L'analyse du produit obtenu donne les résultats suivants :

| Analyse | Calculé pour $C_{14}H_{14}N_2O_3$ | Trouvé |
|---|---|---|
| C | 65,10 | 65,23 |
| H | 5,46 | 5,64 |
| N | 10,85 | 10,73 |
| O | 18,59 | 18,57 |

3ème étape :

Préparation du 5-acétamido 2-(N-benzyloxycarbonyl)amino phénol.

A une solution de 0,6 mole (172 g) de 5-amino 2-(benzyloxycarbonyl)aminophénol dans 465 ml de dioxane chauffé à 70°C, on ajoute goutte à goutte 0,6 mole (57 ml) d'anhydride acétique. Après refroidissement du milieu réactionnel, le produit attendu précipite. Recristallisé de l'éthanol à 96°, il fond à 205°C.

L'analyse du produit obtenu donne les résultats suivants :

| Analyse | Calculé pour $C_{16}H_{16}N_2O_4$ | Trouvé |
|---|---|---|
| C | 63,99 | 63,85 |
| H | 5,37 | 5,52 |
| N | 9,33 | 9,18 |
| O | 21,31 | 21,58 |

4ème étape :

Préparation du chlorhydrate de 5-acétamido 2-aminophénol

On porte au reflux 0,6 mole (182 g) de 5-acétamido 2-(benzyloxycarbonyl)aminophénol dans 910 ml d'éthanol absolu additionné de 310 ml de cyclohexène, en présence de 91 g de palladium à 10% sur charbon (50% humide). Après 1 heure de réaction, le dégagement de gaz carbonique cesse, le catalyseur est éliminé par filtration à chaud. Par ajout de 90 ml d'éthanol chlorhydrique (7M) au filtrat refroidi, on précipite le produit attendu.

L'analyse du produit obtenu donne les résultats suivants :

| Analyse | Calculé pour $C_8H_{11}N_2O_2Cl$ | Trouvé |
|---|---|---|
| C | 47,10 | 47,15 |
| H | 5,43 | 5,49 |
| N | 13,82 | 13,79 |
| O | 15,80 | 15,96 |
| Cl | 17,53 | 17,55 |

EXEMPLE DE PREPARATION 2

Préparation de l'hydrate du chlorhydrate du 2-amino 5-(N-éthoxycarbonyl)aminophénol.

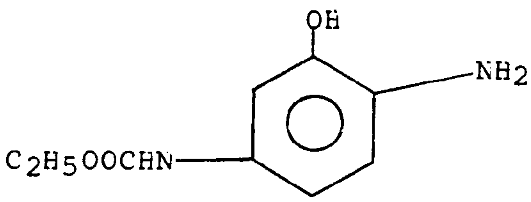

, HCl

1ère étape :

Préparation du 5-(N-éthoxycarbonyl)amino 2-(N-benzyloxy carbonyl)aminophénol.

A 0,13 mole (33 g) de 5-amino 2-(benzyloxy carbonyl)aminophénol (préparé dans l'example 1, 2ème étape) et 7,1 g de carbonate de calcium dans 100 ml de dioxane portés à 80°C, on ajoute, goutte à goutte, lentement, 0,14 mole (15,7 g) de chloroformiate d'éthyle. On chauffe 20 minutes supplémentaires après la fin de l'addition. On filtre à chaud le milieu réactionnel afin d'éliminer les sels minéraux. Par dilution à l'eau glacée du filtrat refroidi, on précipite le produit attendu. Recristallisé de l'acétate d'éthyle, il fond à 190°C.

L'analyse du produit obtenu donne les résultats suivants :

| Analyse | Calculé pour $C_{17}H_{18}N_2O_5$ | Trouvé |
|---|---|---|
| C | 61,81 | 61,71 |
| H | 5,45 | 5,51 |
| N | 8,48 | 8,43 |
| O | 24,24 | 24,49 |

2ème étape :

Préparation du 2-amino 5-(N-éthoxycarbonyl)aminophénol.

On chauffe au reflux pendant 30 minutes 0,06 mole (20 g) de 5-(éthoxycarbonyl)amino 2-(benzyloxy carbonyl)aminophénol dans 60 ml d'éthanol à 96° additionné de 40 ml de cyclohéxène et de 4 g de palladium à 10% sur charbon. Après élimination par filtration à chaud du catalyseur, le produit attendu est précipité par ajout au filtrat refroidi de 17,5 ml d'éthanol chlorhydrique (7M). Après séchage, l'analyse du produit obtenu donne les résultats suivants :

| Analyse | Calculé pour $C_9H_{15}ClN_2O_4$ | Trouvé |
|---|---|---|
| C | 43,12 | 43,20 |
| H | 6,03 | 6,58 |
| N | 11,17 | 11,23 |
| O | 25,53 | 24,67 |
| Cl | 14,14 | 14,20 |

EXEMPLE DE PREPARATION 3

Préparation du chlorhydrate de 2-amino 5-(N-benzyl carbonyl)aminophénol.

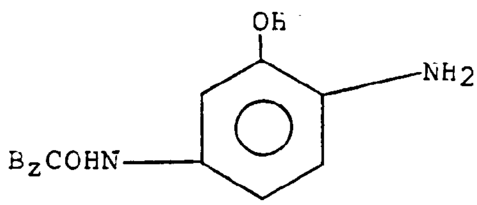

, HCl

1ère étape :

Préparation du 2-(N-benzyloxycarbonyl)amino 5-(N-benzyl carbonyl)aminophénol.

A 0,77 mole (20 g) de 5-amino 2-(benzyloxy carbonyl)aminophénol (préparé à la 2ème étape de l'exemple 1) et 12 g de carbonate de calcium dans 120 ml de dioxane portés à 80°C, on ajoute goutte à goutte lentement 12 g de chlorure de benzoyle. On chauffe 20 minutes supplémentaires après la fin de l'addition. On filtre à chaud afin d'éliminer les sels minéraux. Par dilution du filtrat à l'eau glacée, on précipite le produit attendu. Recristallisé du méthoxyéthanol, il fond à 252°C.

L'analyse du produit obtenu donne les résultats suivants :

| Analyse | Calculé pour $C_{22}H_{20}N_2O_4$ | Trouvé |
|---|---|---|
| C | 69,23 | 69,46 |
| H | 5,49 | 5,54 |
| N | 7,69 | 7,65 |
| O | 17,58 | 17,46 |

2ème étape :

Préparation du chlorhydrate de 2-amino 5-(N-benzyl carbonyl)aminophénol.

On chauffe au reflux pendant 35 minutes 0,058 mole (22 g) de 2-(benzyloxycarbonyl)amino 5-(benzyl carbonyl)aminophénol dans 66 ml d'éthanol à 96° additionnés de 44 ml de cyclohexène et de 4,4 g de palladium à 10% sur charbon. Après élimination par filtration à chaud du catalyseur, on ajoute au filtrat refroidi 30 ml d'éthanol absolu et 17 ml d'éthanol chlorhydrique (7M). Le produit attendu précipite. Après séchage, l'analyse du produit obtenu donne les résultats suivants :

| Analyse | Calculé pour $C_{14}H_{15}N_2O_2Cl$ | Trouvé |
|---|---|---|
| C | 60,32 | 60,21 |
| H | 5,38 | 5,44 |
| N | 10,05 | 10,25 |
| O | 11,49 | 11,66 |
| Cl | 12,75 | 12,70 |

EXEMPLE DE PREPARATION 4

Préparation du chlorhydrate de 5-acétamido 2-(β-hydroxy éthyl)aminophénol.

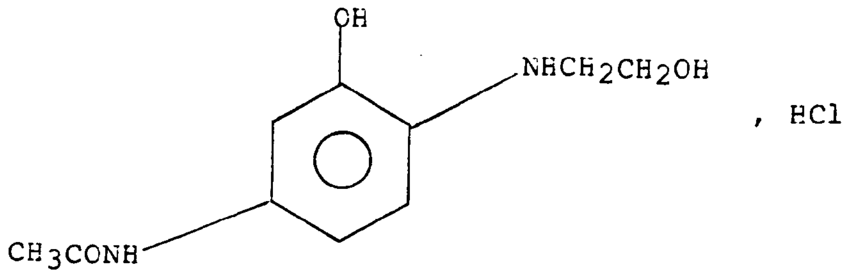

1ère étape :

Préparation du 2-(N-benzyloxycarbonyl N-β-hydroxyéthyl) amino 5-nitrophénol.

A 1,1 mole (220 g) de 2-(β-hydroxyéthyl)amino 5-nitrophénol et 61 g de carbonate de calcium dans 733 ml de dioxane portés à 70°C, on ajoute 11 mole (197,7 g) de chloroformiate de benzyle en maintenant la température entre 70 et 80°C. L'addition terminée, on maintient la température pendant une heure à 80°C, puis on laisse refroidir à la température ambiante. On verse alors le mélange réactionnel sur 10 kg d'eau glacée et on acidifie par un acide fort. Le produit attendu cristallise. Après filtration et séchage, le produit obtenu est recristallisé d'un mélange de cyclohexane et de 1,2-dichloroéthane. Il fond alors à 136°C.

L'analyse du produit obtenu donne les résultats suivants.

Analyse pour $C_{16}H_{16}N_2O_6$

| | C | H | N | O |
|---|---|---|---|---|
| Calculé | 57,83 | 4,82 | 8,43 | 28,91 |
| Trouvé | 57,85 | 4,75 | 8,45 | 29,08 |

2ème étape :

Préparation du 5-amino 2-(N-benzyloxycarbonyl N-β-hydroxyéthyl)aminophénol.

Dans un autoclave, on soumet à une pression d'hydrogène de 9.10$^5$ Pascal (9 bar) 0,24 mole (80 g) de 2-(N-benzyloxycarbonyl N-β-hydroxyéthyl)amino 5-nitro phénol dans 630 ml d'acétate d'éthyle en présence de 15 g de nickel de Raney (50% humide). Après une heure à 85°C, le milieu réactionnel est filtré chaud afin d'éliminer le catalyseur. Par évaporation sous vide du solvant, le produit attendu cristallise. Après essorage et séchage, le produit obtenu est recristallisé de l'alcool à 96°. Il fond à 142°C.

L'analyse du produit obtenu donne les résultats suivants.

Analyse pour $C_{16}H_{18}N_2O_4$

| | C | H | N | O |
|---|---|---|---|---|
| Calculé | 63,57 | 5,96 | 9,27 | 21,19 |
| Trouvé | 63,29 | 5,90 | 9,23 | 21,29 |

3ème étape :

Préparation de l'hémihydrate du 5-acétamido 2-(N-benzyloxycarbonyl N-β-hydroxyéthyl)aminophénol.

A une solution de 0,24 mole (72 g) de 5-amino 2-(N-benzyloxycarbonyl N-β-hydroxyéthyl)aminophénol dans un litre d'acétate d'éthyle chauffé à 80°C, on ajoute goutte à goutte 0,24 mole (23 ml) d'anhydride acétique. Après évaporation sous vide du solvant, le produit attendu est purifié sur colonne de silice. Il fond alors à 72°C.

Analyse pour $C_{18}H_{21}N_2O_{5,5}$

| | C | H | N | O |
|---|---|---|---|---|
| Calculé | 61,18 | 5,94 | 7,9 | 24,92 |
| Trouvé | 61,57 | 5,93 | 7,88 | 24,80 |

4ème étape :

Préparation du chlorhydrate de 5-acétamido 2-(N-β-hydroxyéthyl)aminophénol.

On porte au reflux $5,8.10^{-3}$ mole de 5-acétamido 2-(N-benzyloxycarbonyl N-β-hydroxyéthyl)aminophénol dans 10 ml d'éthanol à 96° additionné de 6 ml de cyclohexène, en présence de 0,35 g de palladium à 10% sur charbon (50% humide). Après une heure de réaction, le dégagement de gaz carbonique cesse, le catalyseur est éliminé par filtration à chaud. Par ajout de 1,35 ml d'éthanol chlorhydrique (7 M) au filtrat refroidi, on précipite le produit attendu.

L'analyse du produit obtenu donne les résultats suivants.

Analyse pour $C_{10}H_{15}N_2O_3Cl$

| | C | H | N | O | Cl |
|---|---|---|---|---|---|
| Calculé | 48,68 | 6,08 | 11,35 | 19,47 | 14,40 |
| Trouvé | 48,67 | 6,15 | 11,20 | 19,68 | 14,30 |

EXEMPLE DE PREPARATION 5

Préparation du chlorhydrate de 5-acétamido 2-(méthyl) aminophénol.

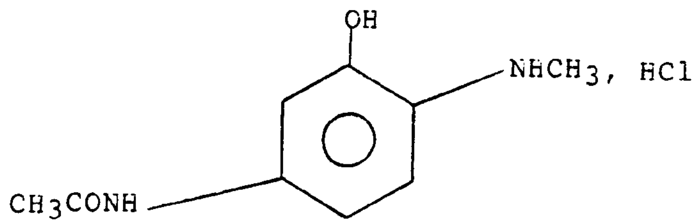

1ère étape :

Préparation du 2-(N-benzyloxycarbonyl N-méthyl)amino 5-nitrophénol.

A 0,24 mole (41,5 g) de 2-méthylamino 5-nitrophénol et de 15,6 g de carbonate de calcium dans 125 ml de dioxane porté à 80°C, on ajoute goutte à goutte 0,26 mole (40 ml) de chloroformiate de benzyle en maintenant la température entre 80°C et 95°C. L'addition terminée, on maintient le chauffage deux heures supplémentaires. Les sels minéraux présents dans le milieu réactionnel sont éliminés par filtration à chaud. Après addition d'eau glacée, le produit attendu cristallise. Le produit obtenu est recristallisé de l'acide acétique. Il fond à 192°C.

L'analyse du produit obtenu donne les résultats suivants.

Analyse pour $C_{15}H_{14}N_2O_5$

| | C | H | N | O |
|---|---|---|---|---|
| Calculé | 59,60 | 4,63 | 9,27 | 26,49 |
| Trouvé | 59,56 | 4,65 | 9,28 | 26,53 |

2ème étape :

Préparation du 5-acétamido 2-(N-benzyloxycarbonyl N-méthyl)aminophénol.

A 40 g de fer en poudre dans 60 ml d'éthanol à 96° mélangés à 20 ml d'acide acétique portés à 80°C, on ajoute $3{,}3.10^{-2}$ mole (10 g) de 2-(N-benzyloxycarbonyl N-méthyl)amino 5-nitrophénol en maintenant la température à 80°C. L'addition terminée, on maintient le chauffage pendant 30 minutes. Les sels minéraux présents dans le milieu réactionnel sont éliminés par filtration à chaud. Le filtrat est dilué par 800 ml d'eau glacée. On ajoute alors $3{,}7.10^{-2}$ mole (3,5 ml) d'anhydride acétique. Le produit attendu cristallise au bout de 3 heures. Après filtration et séchage, le produit obtenu est recristallisé d'un mélange eau/éthanol. Il fond alors à 174°C.

L'analyse du produit obtenu donne les résultats suivants.

Analyse pour $C_{17}H_{18}N_2O_4$

| | C | H | N | O |
|---|---|---|---|---|
| Calculé | 64,96 | 5,73 | 8,91 | 20,38 |
| Trouvé | 64,97 | 5,75 | 8,87 | 20,39 |

3ème étape :

Préparation du chlorhydrate de 5-acétamido 2-(méthyl) aminophénol.

On porte à reflux $5,4.10^{-3}$ mole (1,7 g) de 5-acétamido 2-(N-benzyloxycarbonyl N-méthyl)aminophénol dans 8,5 ml d'éthanol à 96° additionné de 5,1 ml de cyclohéxène en présence de 0,35 g de palladium sur charbon (50% humide). Après une heure de reflux, le dégagement de gaz carbonique cesse, le catalyseur est éliminé par filtration à chaud. Par ajout de 1,6 ml d'éthanol chlorhydrique (7 M) au filtrat refroidi, on précipite le produit attendu.

L'analyse du produit obtenu donne les résultats suivants.

Analyse pour $C_9H_{13}N_2O_2Cl$

| | C | H | N | O | Cl |
|---|---|---|---|---|---|
| Calculé | 49,88 | 6,00 | 12,93 | 14,78 | 16,39 |
| Trouvé | 49,77 | 6,07 | 12,78 | 14,90 | 16,28 |

Les compositions tinctoriales pour fibres kératiniques et en particulier pour cheveux humains, selon l'invention, contiennent, dans un véhicule aqueux, au moins un composé de formule (I).

Les composés de formule (I) sont utilisés dans les compositions de l'invention à des concentrations comprises entre 0,02 et 6% et de préférence comprises entre 0,15 et 5% en poids, par rapport au poids total de la composition.

Les compositions de l'invention peuvent contenir également d'autres précurseurs de colorants par oxydation.

Parmi ces précurseurs de colorants par oxydation, il faut citer les paraphénylènediamines, les para-aminophénols, les orthophénylènediamines et les ortho-aminophénols, les bases hétérocycliques comme la 2,5-diaminopyridine.

Parmi les paraphénylènediamines, il faut citer plus particulièrement :

la paraphénylènediamine,
la paratoluylènediamine,
la 2,6-diméthylparaphénylènediamine,
la 2,6-diméthyl 3-méthoxyparaphénylène diamine,
la N-(β-méthoxyéthyl)paraphénylènediamine,
la 4-N-[β-(β'-hydroxyéthoxy)éthyl]amino aniline,
la 4-N,N-di-(β-hydroxyéthyl)aminoaniline,
la 4-N,N-(éthyl, carbamylméthyl)aminoaniline,
ainsi que leurs sels.

Parmi les para-aminophénols, il faut citer plus particulièrement :

le para-aminophénol,
le 2-méthyl 4-aminophénol,
le 2-chloro 4-aminophénol,
le 2,6-diméthyl 4-aminophénol,
le 2,3-diméthyl 4-aminophénol,
le 2,5-diméthyl 4-aminophénol,
le 2-hydroxyméthyl 4-aminophénol,
le 2-β-hydroxyéthyl 4-aminophénol,
ainsi que leurs sels.

Parmi les orthophénylènediamines et les ortho-aminophénols pouvant être substitués sur le noyau ou sur les fonctions amines, il faut citer en particulier le 1-amino 2-hydroxybenzène, le 6-méthyl 1-hydroxy 2-aminobenzène, le 4-méthyl 1-amino 2-hydroxy benzène.

Un autre objet de l'invention est constitué par les compositions tinctoriales contenant en association avec les composés (I) des coupleurs.

Parmi les coupleurs, on peut citer en particulier les phénols, les métadiphénols, les méta-aminophénols, les métaphénylènediamines, les méta-acylaminophénols, les méta-uréidophénols, les métacarbalcoxyaminophénols, l'α-naphtol, les coupleurs possédant un groupe méthylène actif, tels que les composés β-cétoniques

et les pyrazolones.

Parmi les phénols, on peut citer le 2-isopropyl 5-méthylphénol.

Parmi les métadiphénols, on peut citer :

la résorcine,
la 2-méthylrésorcine,
la 5-méthylrésorcine,
le 2,4-dihydroxyphénoxyéthanol,
le monométhyléther de résorcine,
le 2,4-dihydroxyanisole,

Parmi les méta-aminophénols, on peut citer :

le méta-aminophénol,
le 2-méthyl 5-aminophénol,
le 2-méthyl-5-N-(β-hydroxyéthyl)amino phénol,
le 2-méthyl-5-N-(β-mésylaminoéthyl)amino phénol,
le 2,6-diméthyl 3-aminophénol,
la 6-hydroxybenzomorpholine,
et leurs sels.

Parmi les métaphénylènediamines, on peut citer :

la métaphénylènediamine,
le 2,4-diaminophénoxyéthanol,
le 2,4-diméthoxy 1,3-diaminobenzène,
le 1,3,5-triméthoxy 2,4-diaminobenzène
le 2,4-diaminoanisole,
la 6-aminobenzomorpholine,
le [2-N-(β-hydroxyéthyl)amino 4-amino] phénoxyéthanol,
le [4-N-(β-hydroxyéthyl)amino 2-amino] phénoxyéthanol,
le 2-amino 4-N-(β-hydroxyéthyl)aminoanisole,
le di-4,5-(β-hydroxyéthoxy)1,3-diamino benzène,
le 1-β-hydroxyéthoxy 2,4-diaminobenzène,
et leurs sels.

Parmi les autres coupleurs utilisables dans les compositions tinctoriales de l'invention, il faut citer plus particulièrement :

le 3,4-méthylènedioxyphénol,
la 3,4-méthylènedioxyaniline,
le 2-bromo 4,5-méthylènedioxyphénol,
le 2-chloro 4,5-méthylènedioxyphénol,
la 2-méthoxy 4,5-méthylènedioxyaniline.

Les compositions tinctoriales selon l'invention peuvent contenir en association avec les composés de formule (I), les précurseurs de colorants d'oxydation et les coupleurs définis ci-dessus.

Le poids total des précurseurs de colorants d'oxydation et/ou des coupleurs utilisés dans les compositions tinctoriales selon l'invention est de préférence de 0,1 à 7% en poids du poids total de la composition tinctoriale.

Les compositions tinctoriales selon l'invention peuvent également renfermer des colorants directs tels que des colorants azoïques, anthraquinoniques et les dérivés nitrés de la série benzénique, qui permettent de nuancer ou d'enrichir en reflets les colorations apportées par les précurseurs de colorants d'oxydation et/ou les coupleurs.

Le pH de la composition tinctoriale est généralement compris entre 8 et 11 et de préférence entre 9 et 11. Ce pH est ajusté à la valeur désirée à l'aide d'un agent alcalinisant tel que l'ammoniaque, les carbonates alcalins, les alcanolamines telles que la mono-, la di- ou la triéthanolamine.

Les compositions tinctoriales conformes à l'invention contiennent dans leur forme de réalisation préférée des agents tensio-actifs anioniques, cationiques, non-ioniques, amphotères ou leurs mélanges. Ces agents tensio-actifs sont présents dans les compositions conformes à l'invention dans des proportions comprises entre 0,5 et 40% en poids, et de préférence entre 2 et 30% en poids par rapport au poids total de la composition.

Ces compositions peuvent également contenir des solvants organiques pour solubiliser les composés qui ne seraient par suffisamment solubles dans l'eau. Parmi ces agents, on peut mentionner à titre d'exemple, les alcools en $C_1$-$C_8$, tels que l'éthanol, l'isopropanol, l'alcool benzylique et l'alcool phényléthylique, le glycérol; les glycols ou éthers de glycol comme le butoxy-2 éthanol, l'éthylèneglycol, le propylèneglycol, le monoéthyléther et le monométhyléther du diéthylène glycol, ainsi que les produits analogues et leurs mélanges. Les solvants sont présents de préférence dans une proportion comprise entre 1 et 40% en poids, et en particulier

entre 2 et 30% en poids par rapport au poids total de la composition.

Les agents épaississants que l'on peut ajouter dans les compositions conformes à l'invention sont pris notamment dans le groupe formé par l'alginate de sodium, la gomme arabique, les dérivés de la cellulose tels que la méthylcellulose, l'hydroxyéthylcellulose, l'hydroxypropylcellulose, l'hydroxyméthylcellulose, la carboxyméthylcellulose, les polymères d'acide acrylique, la gomme de xanthane. On peut également utiliser des agents épaississants minéraux tels que la bentonite. Ces agents épaississants sont présents de préférence en des proportions comprises entre 0,1 et 5% en poids, et en particulier entre 0,5 et 3% en poids du poids total de la composition.

Les compositions peuvent contenir des agents antioxydants choisis en particulier parmi le sulfite de sodium, l'acide thioglycolique, le bisulfite de sodium, l'acide ascorbique et l'hydroquinone. Ces agents antioxydants sont présents dans la composition dans des proportions comprises entre 0,05 et 1,5% en poids du poids total de la composition.

D'autres adjuvants utilisables conformément à l'invention sont par exemple des agents de pénétration, des agents séquestrants, des tampons et des parfums.

Les compositions tinctoriales conformes à l'invention peuvent se présenter sous des formes diverses, notamment sous forme de liquides, de crèmes, de gels et sous toute autre forme appropriée, pour réaliser une teinture des fibres kératiniques et notamment des cheveux humains. Elles peuvent également être conditionnées en flacons aérosols en présence d'un agent propulseur.

Les compositions tinctoriales selon l'invention sont utilisées dans un procédé de teinture des cheveux mettant en oeuvre la révélation par un oxydant.

Conformément à ce procédé, on mélange au moment de l'emploi la composition tinctoriale décrite ci-dessus avec une solution oxydante, en une quantité suffisante pour oxyder les précurseurs de colorants d'oxydation, ensuite on applique sur les cheveux le mélange obtenu.

La solution oxydante contient en solution aqueuse des agents d'oxydation choisis dans le groupe formé par l'eau oxygénée, le peroxyde d'urée et les persels, tels que le persulfate d'ammonium. On utilise de préférence une solution d'eau oxygénée à 6% en poids (20 volumes).

Selon le procédé de teinture généralement utilisé, le mélange obtenu est appliqué sur les cheveux, à la température ambiante ou à une température ne dépassant pas 40°C, on le laisse poser pendant 10 à 40 minutes, et de préférence pendant 15 à 30 minutes, après quoi on rince les cheveux, on les lave au shampooing, on les rince à nouveau et on les sèche.

Un autre procédé de mise en oeuvre du précurseur de colorant d'oxydation de formule (I) conforme à l'invention, consiste à teindre les cheveux suivant un procédé en plusieurs temps, selon lequel, dans un premier temps, on applique le précurseur de colorant d'oxydation de formule (I) en présence éventuellement d'autres précurseurs, au moyen d'une composition susdéfinie et dans un second temps, on applique le ou les coupleurs. L'agent oxydant est présent dans la composition appliquée dans le second temps ou bien appliqué sur les cheveux eux-mêmes dans un troisième temps, chacune des compositions appliquées en premier et second temps et, s'il y a lieu, l'agent oxydant appliqué dans un troisième temps, sont laissés au contact des cheveux pendant 10 à 40 minutes et de préférence pendant 15 à 30 minutes, après quoi on rince les cheveux, on les lave au shampooing, on les rince à nouveau et on les sèche.

L'invention est illustrée par les exemples d'application ci-après.

EXEMPLES D'APPLICATION

EXEMPLE 1

On prépare le mélange tinctorial suivant :

| | | | |
|---|---|---|---|
| - 2-amino 5-acétamidophénol | | 0,5 | g |
| - Hydroxyéthylcellulose vendue sous la dénomination CELLOSIZE WP 03 par UNION CARBIDE | | 2,0 | g |
| - Laurylsulfate d'ammonium | | 5,0 | g |
| - 2-butoxyéthanol | | 15,0 | g |
| - Ethanol à 96° | | 6,0 | g |
| - Thiolactate d'ammonium | | 0,8 | g |
| - Ammoniaque à 22° Bé | | 10,0 | g |
| - Eau | qsp | 100,0 | g |
| - pH = 10,4 | | | |

Au moment de l'emploi, on ajoute 80 g d'eau oxygénée à 20 volumes (6% en poids). Le mélange, appliqué 15 minutes à 35°C sur cheveux naturellement blancs à 90% leur confère, après shampooing et rinçage, une coloration beige rosé.

EXEMPLE 2

On prépare le mélange tinctorial suivant :

| | | | |
|---|---|---|---|
| - 2-amino 5-(éthoxycarbonyl)aminophénol | | 0,55 | g |
| - Acide polyacrylique réticulé, vendu sous la dénomination CARBOPOL 934 par GOODRICH CHEMICAL | | 3,0 | g |
| - Ethanol à 96° | | 11,0 | g |
| - 2-butoxyéthanol | | 5,0 | g |
| - Bromure de triméthylcétylammonium | | 2,0 | g |
| - Acide éthylènediamine tétracétique, vendu sous la dénomination TRILON B | | 0,2 | g |
| - Ammoniaque à 22° Bé | | 10,0 | g |
| - Solution de bisulfite de sodium à 35° Bé | | 1,0 | g |
| - Eau | qsp | 100,0 | g |
| - pH = 9,1 | | | |

Au moment de l'emploi, on ajoute 70 g d'eau oxygénée à 20 volumes. Le mélange, appliqué 20 minutes à 35°C sur cheveux décolorés leur confère, après shampooing et rinçage, une coloration beige cendré.

EXEMPLE 3

On prépare le mélange tinctorial suivant :

| | | | |
|---|---|---|---|
| - 2-amino 5-(benzylcarbonyl)aminophénol | | 0,69 | g |
| - Acide polyacrylique réticulé, vendu sous la dénomination CARBOPOL 934 par GOODRICH CHEMICAL | | 3,0 | g |
| - Ethanol à 96° | | 11,0 | g |
| - 2-butoxyéthanol | | 5,0 | g |
| - Bromure de triméthylcétylammonium | | 2,0 | g |
| - Acide éthylènediamine tétracétique, vendu sous la dénomination TRILON B | | 0,2 | g |
| - Ammoniaque à 22° Bé | | 10,0 | g |
| - Solution de bisulfite de sodium à 35° Bé | | 1,0 | g |
| - Eau | qsp | 100,0 | g |
| - pH = 9,1 | | | |

Au moment de l'emploi, on ajoute 100 g d'eau oxygénée à 20 volumes. Le mélange, appliqué 20 minutes à 35°C sur cheveux décolorés leur confère, après shampooing et rinçage, une coloration rose foncé gris.

EXEMPLE 4

On prépare le mélange tinctorial suivant :

| | | |
|---|---|---|
| - 2-amino 5-acétamidophénol | | 0,5 g |
| - p-phénylènediamine | | 0,27 g |
| - Alcool oléique polyglycérolé à 2 moles de glycérol | | 4,5 g |
| - Alcool oléique polyglycérolé à 4 moles de glycérol | | 4,5 g |
| - Oléylamine oxyéthylénée à 12 moles d'oxyde d'éthylène, vendue sous la dénomination ETHOMEEN O 12 par ARMOON HESS CHEMICAL Ltd | | 4,5 g |
| - Diéthanolamide de coprah, vendu sous la dénomination COMPERLAN KD par HENKEL | | 9,0 g |
| - Propylèneglycol | | 4,0 g |
| - 2-butoxyéthanol | | 8,0 g |
| - Ethanol à 96° | | 6,0 g |
| - Sel pentasodique de l'acide diéthylène triamine pentacétique, vendu sous la dénomination MASQUOL DTPA par PROTEX | | 2,0 g |
| - Hydroquinone | | 0,15 g |
| - Solution de bisulfite de sodium à 35° Bé | | 1,3 g |
| - Ammoniaque à 22° Bé | | 10,0 g |
| - Eau | qsp | 100,0 g |
| - pH = 10 | | |

Au moment de l'emploi, on ajoute 100 g d'eau oxygénée à 20 volumes. Le mélange, appliqué 20 minutes à 35°C sur cheveux naturellement blancs à 90% leur confère, après shampooing et rinçage, une coloration pourpre gris foncé.

EXEMPLE 5

On prépare le mélange tinctorial suivant :

| | | | |
|---|---|---|---|
| - 2-amino 5-acétamidophénol | | 0,55 | g |
| - p-phénylènediamine | | 0,27 | g |
| - Hydroxyéthylcellulose vendue sous la dénomination CELLOSIZE WP 03 par UNION CARBIDE | | 2,0 | g |
| - Laurylsulfate d'ammonium | | 5,0 | g |
| - 2-butoxyéthanol | | 15,0 | g |
| - Ethanol à 96° | | 6,0 | g |
| - Thiolactate d'ammonium | | 0,8 | g |
| - Ammoniaque à 22° Bé | | 10,0 | g |
| - Eau | qsp | 100,0 | g |
| - pH = 10,4 | | | |

Au moment de l'emploi, on ajoute 100 g d'eau oxygénée à 20 volumes. Le mélange, appliqué 20 minutes à 35°C sur cheveux naturellement blancs à 90% leur confère, après shampooing et rinçage, une coloration violine.

EXEMPLE 6

On prépare le mélange tinctorial suivant :

| | | | |
|---|---|---|---|
| - 2-amino 5-(benzylcarbonyl)aminophénol | | 0,69 | g |
| - 4-amino 2-hydroxyméthylphénol | | 0,35 | g |
| - Acide polyacrylique réticulé, vendu sous la dénomination CARBOPOL 934 par GOODRICH CHEMICAL | | 3,0 | g |
| - Ethanol à 96° | | 11,0 | g |
| - 2-butoxyéthanol | | 5,0 | g |
| - Bromure de triméthylcétylammonium | | 2,0 | g |
| - Acide éthylènediamine tétracétique, vendu sous la dénomination TRILON B | | 0,2 | g |
| - Ammoniaque à 22° Bé | | 10,0 | g |
| - Solution de bisulfite de sodium à 35° Bé | | 1,0 | g |
| - Eau | qsp | 100,0 | g |
| - pH = 9 | | | |

Au moment de l'emploi, on ajoute 90 g d'eau oxygénée à 20 volumes. Le mélange, appliqué 15 minutes à 35°C sur cheveux décolorés leur confère, après shampooing et rinçage, une coloration vieux rose.

EXEMPLE 7

On prépare le mélange tinctorial suivant :

| | | | |
|---|---|---|---|
| - 2-amino 5-(éthoxycarbonyl)aminophénol | | 0,55 | g |
| - Dichlorhydrate de N,N-di-(ß-hydroxyéthyl) 4-amino aniline | | 0,67 | g |
| - Nonylphénol oxyéthylèné à 4 moles d'oxyde d'éthylène, vendu sous la dénomination CEMULSOL NP 4 par RHONE POULENC | | 12,0 | g |
| - Nonylphénol oxyéthylèné à 9 moles d'oxyde d'éthylène, vendu sous la dénomination CEMULSOL NP 9 par RHONE POULENC | | 15,0 | g |
| - Alcool oléique polyglycérolé à 2 moles de glycérol | | 1,5 | g |
| - Alcool oléique polyglycérolé à 4 moles de glycérol | | 1,5 | g |
| - Propylèneglycol | | 6,0 | g |
| - Acide éthylènediamine tétracétique, vendu sous la dénomination TRILON B | | 0,12 | g |
| - Ammoniaque à 22° Bé | | 11,0 | g |
| - Eau | qsp | 100,0 | g |
| - pH = 10,1 | | | |

Au moment de l'emploi, on ajoute 100 g d'eau oxygénée à 20 volumes. Le mélange, appliqué 20 minutes à 35°C sur cheveux décolorés leur confère, après shampooing et rinçage, une coloration bleu pervenche.

EXEMPLE 8

On prépare le mélange tinctorial suivant :

| | | | |
|---|---|---|---|
| - 2-amino 5-(éthoxycarbonyl)aminophénol | | 0,55 | g |
| - 4-amino 2-méthylphénol | | 0,31 | g |
| - Hydroxyéthylcellulose, vendue sous la dénomination CELLOSIZE WP 03 par UNION CARBIDE | | 2,0 | g |
| - Laurylsulfate d'ammonium | | 5,0 | g |
| - 2-butoxyéthanol | | 15,0 | g |
| - Ethanol à 96° | | 6,0 | g |
| - Ammoniaque à 22° Bé | | 10,0 | g |
| - Solution de bisulfite de sodium à 35° Bé | | 1,5 | g |
| - Hydroquinone | | 0,15 | g |
| - Eau | qsp | 100,0 | g |
| - pH = 10 | | | |

Au moment de l'emploi, on ajoute 100 g d'eau oxygénée à 20 volumes. Le mélange, appliqué 20 minutes à 35°C sur cheveux naturellement blancs à 90% leur confère, après shampooing et séchage, une coloration beige rosé.

EXEMPLE 9

On prépare le mélange tinctorial suivant :

| | | |
|---|---|---|
| - 2-amino 5-(benzylcarbonyl)aminophénol | 0,69 | g |
| - Dichlorhydrate de 4-amino 1-(ß-méthoxy-éthyl)aminobenzène | 0,60 | g |
| - Alcool cétylstéarylique vendu sous la dénomination ALFOL C 16/18 par CONDEA | 19,0 | g |
| - 2-octyldodécanol vendu sous la dénomination EUTANOL G par HENKEL | 4,5 | g |
| - Alcool cétylstéarylique à 15 moles d'oxyde d'éthylène, vendu sous la dénomination MERGITAL C.S. par HENKEL | 2,5 | g |
| - Laurylsulfate d'ammonium | 10,0 | g |
| - Polymère cationique présentant le motif récurrent suivant : | 4,0 | g |

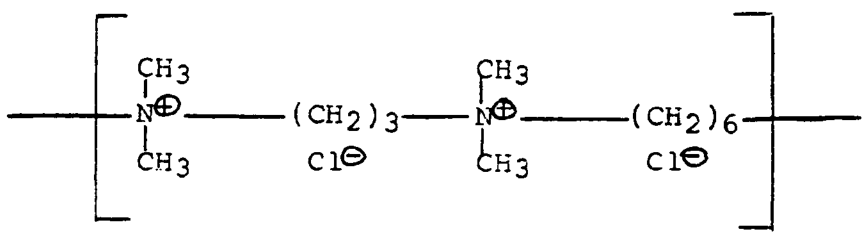

| | | | |
|---|---|---|---|
| - Alcool benzylique | | 2,0 | g |
| - Ammoniaque à 22° Bé | | 11 | ml |
| - Acide éthylènediamine tétracétique, vendu sous la dénomination TRILON B | | 1,0 | g |
| - Solution de bisulfite de sodium à 35° Bé | | 1,2 | g |
| - Eau | qsp | 100,0 | g |
| - pH = 10,2 | | | |

Au moment de l'emploi, on ajoute 100 g d'eau oxygénée à 20 volumes. Le mélange, appliqué 20 minutes à 35°C sur cheveux naturellement blancs à 90% leur confère, après shampooing et rinçage, une coloration bleu marine.

EXEMPLE 10

On prépare le mélange tinctorial suivant :

| | | | |
|---|---|---|---|
| - 2-amino 5-acétamidophénol | | 0,506 | g |
| - Dichlorhydrate de (2,4-diaminophénoxy) éthanol | | 0,602 | g |
| - Alcool cétylstéarylique vendu sous la dénomination ALFOL C 16/18 par CONDEA | | 8,0 | g |
| - Sulfate cétylstéarylique de sodium vendu sous la dénomination CIRE DE LANETTE E par HENKEL | | 0,5 | g |
| - Huile de ricin éthoxylée vendue sous la dénomination CEMULSOL B par RHONE POULENC | | 1,0 | g |
| - Diéthanolamide oléique | | 1,5 | g |
| - Sel pentasodique de l'acide diéthylène triamine pentacétique, vendu sous la dénomination MASQUOL DTPA par PROTEX | | 2,5 | g |
| - Ammoniaque à 22° Bé | | 11,0 | g |
| - Eau | qsp | 100,0 | g |
| - pH = 10,4 | | | |

Au moment de l'emploi, on ajoute 100 g d'eau oxygénée à 20 volumes. Le mélange, appliqué 20 minutes à 35°C sur cheveux naturellement blancs à 90% leur confère, après shampooing et rinçage, une coloration sable doré.

EXEMPLE 11

On prépare le mélange tinctorial suivant :

| | | | |
|---|---|---|---|
| - 2-amino 5-acétamidophénol | | 0,51 | g |
| - 5-amino 2-méthylphénol | | 0,31 | g |
| - Alcool oléique polyglycérolé à 2 moles de glycérol | | 4,5 | g |
| - Alcool oléique polyglycérolé à 4 moles de glycérol | | 4,5 | g |
| - Oléylamine oxyéthylénée à 12 moles d'oxyde d'éthylène, vendue sous la dénomination ETHOMEEN O 12 par ARMOON HESS CHEMICAL Ltd | | 4,5 | g |
| - Diéthanolamide de coprah, vendu sous la dénomination COMPERLAN KD par HENKEL | | 9,0 | g |
| - Propylèneglycol | | 4,0 | g |
| - 2-butoxyéthanol | | 8,0 | g |
| - Ethanol à 96° | | 6,0 | g |
| - Sel pentasodique de l'acide diéthylène triamine pentacétique, vendu sous la dénomination MASQUOL DTPA par PROTEX | | 2,0 | g |
| - Hydroquinone | | 0,15 | g |
| - Solution de bisulfite de sodium à 35° Bé | | 1,3 | g |
| - Ammoniaque à 22° Bé | | 10,0 | g |
| - Eau | qsp | 100,0 | g |
| - pH = 10 | | | |

Au moment de l'emploi, on ajoute 100 g d'eau oxygénée à 20 volumes. Le mélange, appliqué 20 minutes à 35°C sur cheveux décolorés leur confère, après shampooing et rinçage, une coloration saumon orangé.

EXEMPLE 12

On prépare le mélange tinctorial suivant :

| | | | |
|---|---|---|---|
| - 2-amino 5-acétamidophénol | | 0,40 | g |
| - p-aminophénol | | 0,70 | g |
| - Dichlorhydrate de 4-amino 1-(β-méthoxy-éthyl)aminobenzène | | 1,21 | g |
| - Dichlorhydrate de (2,4-diaminophénoxy) éthanol | | 0,1 | g |
| - 6-hydroxybenzomorpholine | | 0,205 | g |
| - Nonylphénol oxyéthyléné à 4 moles d'oxyde d'éthylène, vendu sous la dénomination CEMULSOL NP 4 par RHONE POULENC | | 12,0 | g |
| - Nonylphénol oxyéthyléné à 9 moles d'oxyde d'éthylène, vendu sous la dénomination CEMULSOL NP 9 par RHONE POULENC | | 15,0 | g |
| - Alcool oléique polyglycérolé à 2 moles de glycérol | | 1,5 | g |
| - Alcool oléique polyglycérolé à 4 moles de glycérol | | 1,5 | g |
| - Propylèneglycol | | 6,0 | g |
| - Acide éthylènediamine tétracétique, vendu sous la dénomination TRILON B | | 0,12 | g |
| - Ammoniaque à 22° Bé | | 11,0 | g |
| - Eau | qsp | 100,0 | g |
| - pH = 10,1 | | | |

Au moment de l'emploi, on ajoute 90 g d'eau oxygénée à 20 volumes. Le mélange, appliqué 25 minutes à 35°C sur cheveux naturellement blancs à 90% leur confère, après shampooing et rinçage, une coloration gris anthracite.

EXEMPLE 13

On prépare le mélange tinctorial suivant :

| | | |
|---|---|---|
| - 2-amino 5-(éthoxycarbonyl)aminophénol | | 0,55 g |
| - 2-bromo 4,5-méthylènedioxyphénol | | 0,54 g |
| - Hydroxyéthylcellulose vendue sous la dénomination CELLOSIZE WP 03 par UNION CARBIDE | | 2,0 g |
| - Laurylsulfate d'ammonium | | 5,0 g |
| - 2-butoxyéthanol | | 15,0 g |
| - Ethanol à 96° | | 5,0 g |
| - Sel pentasodique de l'acide diéthylène triamine pentacétique, vendu sous la dénomination MASQUOL DTPA par PROTEX | | 2,0 g |
| - Triéthanolamine | | 5,0 g |
| - Eau | qsp | 100,0 g |
| - pH = 9 | | |

Au moment de l'emploi, on ajoute 100 g d'eau oxygénée à 20 volumes. Le mélange, appliqué 15 minutes à 35°C sur cheveux décolorés leur confère, après shampooing et rinçage, une coloration blond doré.

EXEMPLE 14

On prépare le mélange tinctorial suivant :

| | | |
|---|---|---|
| - 2-amino 5-(benzylcarbonyl)aminophénol | | 0,69 g |
| - Dichlorhydrate de 2,5-diaminopyridine | | 0,69 g |
| - Nonylphénol oxyéthyléné à 4 moles d'oxyde d'éthylène, vendu sous la dénomination CEMULSOL NP 4 par RHONE POULENC | | 12,0 g |
| - Nonylphénol oxyéthyléné à 9 moles d'oxyde d'éthylène, vendu sous la dénomination CEMULSOL NP 9 par RHONE POULENC | | 15,0 g |
| - Alcool oléique polyglycérolé à 2 moles de glycérol | | 1,5 g |
| - Alcool oléique polyglycérolé à 4 moles de glycérol | | 1,5 g |
| - Propylèneglycol | | 6,0 g |
| - Acide éthylènediamine tétracétique, vendu sous la dénomination TRILON B | | 0,12 g |
| - Ammoniaque à 22° Bé | | 11,0 g |
| - Eau | qsp | 100,0 g |
| - pH = 10,3 | | |

Au moment de l'emploi, on ajoute 100 g d'eau oxygénée à 20 volumes. Le mélange, appliqué 25 minutes à 35°C sur cheveux naturellement blancs à 90% leur confère, après shampooing et rinçage, une coloration châtain clair cuivré.

EXEMPLE 15

On prépare le mélange tinctorial suivant :

| | | | |
|---|---|---|---|
| - 2-amino 5-acétamidophénol | | 0,50 | g |
| - o-aminophénol | | 0,27 | g |
| - Nonylphénol oxyéthyléné à 4 moles d'oxyde d'éthylène, vendu sous la dénomination CEMULSOL NP 4 par RHONE POULENC | | 12,0 | g |
| - Nonylphénol oxyéthyléné à 9 moles d'oxyde d'éthylène, vendu sous la dénomination CEMULSOL NP 9 par RHONE POULENC | | 15,0 | g |
| - Alcool oléique polyglycérolé à 2 moles de glycérol | | 1,5 | g |
| - Alcool oléique polyglycérolé à 4 moles de glycérol | | 1,5 | g |
| - Propylèneglycol | | 6,0 | g |
| - Acide éthylènediamine tétracétique, vendu sous la dénomination TRILON B | | 0,12 | g |
| - Ammoniaque à 22° Bé | | 11,0 | g |
| - Acide thioglycolique | | 0,6 | g |
| - Eau | qsp | 100,0 | g |
| - pH = 10,3 | | | |

Au moment de l'emploi, on ajoute 80 g d'eau oxygénée à 20 volumes. Le mélange, appliqué 15 minutes à 35°C sur cheveux naturellement blancs à 90% leur confère, après shampooing et rinçage, une coloration châtain clair doré.

EXEMPLE 16

On prépare le mélange tinctorial suivant :

| | | |
|---|---|---|
| - Chlorhydrate de 5-acétamido 2-ß-hydroxyéthylaminophénol | | 0,4 g |
| - p-phénylènediamine | | 0,216 g |
| - Alcool oléique polyglycérolé à 2 moles de glycérol | | 4,5 g |
| - Alcool oléique polyglycérolé à 4 moles de glycérol | | 4,5 g |
| - Oléylamine oxyéthylénée à 12 moles d'oxyde d'éthylène, vendue sous la dénomination ETHOMEEN O 12 par ARMOON HESS CHEMICAL Ltd | | 4,5 g |
| - Diéthanolamide de coprah, vendu sous la dénomination COMPERLAN KD PAR HENKEL | | 9,0 g |
| - Propylèneglycol | | 4,0 g |
| - 2-butoxyéthanol | | 8,0 g |
| - Ethanol à 96° | | 6,0 g |
| - Sel pentasodique de l'acide diéthylène triamine pentacétique, vendu sous la dénomination MASQUOL DTPA par PROTEX | | 2,0 g |
| - Hydroquinone | | 0,15 g |
| - Solution de bisulfite de sodium à 35° Bé | | 1,3 g |
| - Ammoniaque à 22° Bé | | 10,0 g |
| - Eau | qsp | 100,0 g |
| - pH = 10 | | |

Au moment de l'emploi, on ajoute 100 g d'eau oxygénée à 20 volumes. Le mélange, appliqué 20 minutes à 35°C sur cheveux naturellement blancs à 90% leur confère, après shampooing et rinçage, une coloration gris violine.

EXEMPLE 17

On prépare le mélange tinctorial suivant :

| | | | |
|---|---|---|---|
| - Chlorhydrate de 5-acétamido 2-méthylaminophénol | | 0,599 | g |
| - p-phénylènediamine | | 0,27 | g |
| - Hydroxyéthylcellulose vendue sous la dénomination CELLOSIZE WP 03 par UNION CARBIDE | | 2,0 | g |
| - Laurylsulfate d'ammonium | | 5,0 | g |
| - 2-butoxyéthanol | | 15,0 | g |
| - Ethanol à 96° | | 6,0 | g |
| - Thiolactate d'ammonium | | 0,8 | g |
| - Ammoniaque à 22° Bé | | 10,0 | g |
| - Eau | qsp | 100,0 | g |
| - pH = 10,4 | | | |

Au moment de l'emploi, on ajoute 100 g d'eau oxygénée à 20 volumes. Le mélange, appliqué 20 minutes à 35°C sur cheveux naturellement blancs à 90% leur confère, après shampooing et rinçage, une coloration violine.

## Revendications

### Revendications pour les Etats contractants suivants : AT, BE, CH, DE, FR, GB, GR, IT, LI, NL, SE

1. Ortho-aminophénols 5-substitués, de formule :

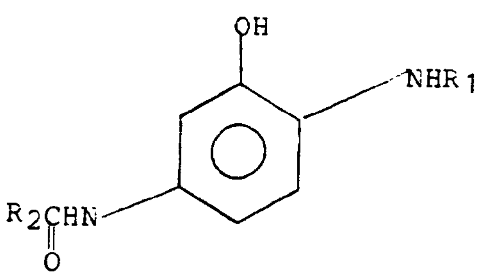

(I)

dans laquelle :

$R_1$ représente un atome d'hydrogène, un radical alkyle ayant 1 à 4 atomes de carbone, un radical hydroxyalkyle ayant 1 à 4 atomes de carbone;

$R_2$ représente, indépendamment de $R_1$, un radical alkyle ayant 1 à 4 atomes de carbone, un radical alcoxy ayant 1 à 4 atomes de carbone, un radical benzyle;

leurs sels d'addition avec un acide et leurs phénates.

2. Ortho-aminophénols 5-substitués, selon la revendication 1, choisis parmi le 5-acétamido 2-amino phénol, le 2-amino 5-(éthoxycarbonyl)aminophénol, le 2-amino 5-(benzylcarbonyl)aminophénol, le 5-acétamido 2-(N-β-hydroxyéthyl)aminophénol, le 5-acétamido 2-(méthyl)aminophénol, et leurs sels.

3. Composition tinctoriale pour fibres kératiniques, en particulier pour cheveux humains, caractérisée par le fait qu'elle contient au moins un ortho-aminophénol 5-substitué, de formule :

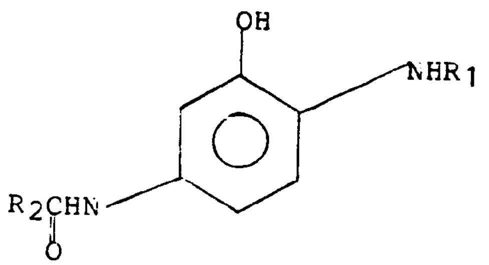

(I)

dans laquelle :

$R_1$ représente un atome d'hydrogène, un radical alkyle ayant 1 à 4 atomes de carbone, un radical hydroxyalkyle ayant 1 à 4 atomes de carbone;

$R_2$ représente, indépendamment de $R_1$, un radical alkyle ayant 1 à 4 atomes de carbone, un radical alcoxy ayant 1 à 4 atomes de carbone, un radical benzyle;

ou un sel d'addition avec un acide ou un phénate d'un composé de formule (I).

**4.** Composition tinctoriale selon la revendication 3, renfermant également un précurseur de colorant d'oxydation.

**5.** Composition tinctoriale selon la revendication 4, dans laquelle les précurseurs de colorants d'oxydation sont choisis parmi les paraphénylènediamines, les para-aminophénols, les orthophénylènediamines, les ortho-aminophénols, les bases hétérocycliques comme la 2,5-diaminopyridine.

**6.** Composition tinctoriale selon les revendications 3 à 5, renfermant également un ou plusieurs coupleurs.

**7.** Composition tinctoriale selon la revendication 6, dans laquelle les coupleurs sont choisis parmi les phénols, les métadiphénols, les méta-aminophénols, les métaphénylènediamines, les méta-acylaminophénols, les méta-uréidophénols, les métacarbalcoxyaminophénols, l' α-naphtol et les coupleurs possédant un groupe méthylène actif tel que les composés β-cétoniques et les pyrazolones; le 3,4-méthylènedioxyphénol, la3,4-méthylènedioxyaniline, le 2-bromo 4,5-méthylènedioxyphénol, le 2-chloro 4,5-méthylènedioxyphénol, la 2-méthoxy, 4,5-méthylènedioxy aniline.

**8.** Composition tinctoriale selon les revendications 3 à 7, dans laquelle les ortho-aminophénols 5-substitués de formule (I) sont utilisés dans des concentrations comprises entre 0,02 et 6% et de préférence entre 0,15 et 5% en poids par rapport au poids total de la composition.

**9.** Composition tinctoriale selon les revendications 3 à 8, dans laquelle le poids total des précurseurs de colorants d'oxydation et des coupleurs est de 0,1 à 7% en poids par rapport au poids total de la composition.

**10.** Composition tinctoriale selon les revendications 3 à 9, dont le pH est compris entre 8 et 11 et de préférence entre 9 et 11.

**11.** Composition tinctoriale selon les revendications 3 à 10, renfermant également des adjuvants choisis dans le groupe formé par les agents tensio-actifs anioniques, cationiques, non-ioniques, amphotères et leurs mélanges, les solvants organiques, les agents épaississants, les agents antioxydants, les séquestrants, les tampons et les parfums.

**12.** Composition tinctoriale selon les revendications 3 à 11, se présentant sous forme de liquide, de crème, de gel ou bien est conditionnée en flacon aérosol en présence d'un agent propulseur.

**13.** Procédé de teinture des cheveux, caractérisé par le fait qu'il consiste, après avoir mélangé la composition tinctoriale telle que définie dans les revendications 3 à 12, avec l'agent oxydant choisi parmi l'eau oxygénée, le peroxyde d'urée et des persels, à appliquer le mélange résultant sur les cheveux pendant une durée de pose de 10 à 40 minutes, à une température ne dépassant pas 40°C, après quoi on rince les cheveux, on les lave au shampooing, on les rince à nouveau et on les sèche.

**14.** Procédé de teinture des cheveux, caractérisé par le fait que l'on applique sur les cheveux, dans un premier temps, une composition tinctoriale selon les revendications 3, 8 et 10 à 12, contenant comme précurseur

de colorant d'oxydation, uniquement des composés de formule (I) ou également d'autres précurseurs de colorants d'oxydation, et dans un second temps, on applique le ou les coupleur(s), l'agent oxydant étant présent dans la composition appliquée dans le second temps ou bien est appliqué sur les cheveux dans un troisième temps, chacune des compositions appliquées en premier et en second temps et, s'il y a lieu, l'agent oxydant appliqué en un troisième temps, sont laissés sur les cheveux pendant 10 à 40 minutes, à une température ne dépassant pas 40°C, après quoi on rince les cheveux, on les lave au shampooing, on les rince à nouveau et on les sèche.

**15.** Procédé de préparation des ortho-aminophénol 5-substitués, de formule :

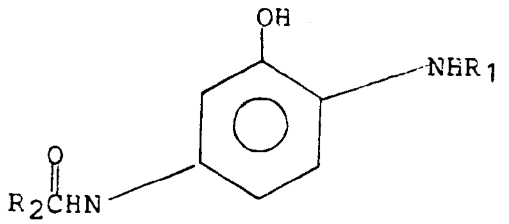

(I)

selon la revendication 1,
où $R_1$ représente un atome d'hydrogène, un radical alkyle ayant de 1 à 4 atomes de carbone, un radical hydroxyalkyle ayant de 1 à 4 atomes de carbone;
$R_2$ représente, indépendamment de $R_1$, un radical alkyle ayant de 1 à 4 atomes de carbone, un radical alcoxy ayant de 1 à 4 atomes de carbone ou un radical benzyle consistant :

- en une première étape, à condenser du chloroformiate de benzyle sur l'aminonitrophénol de formule (V), selon la réaction :

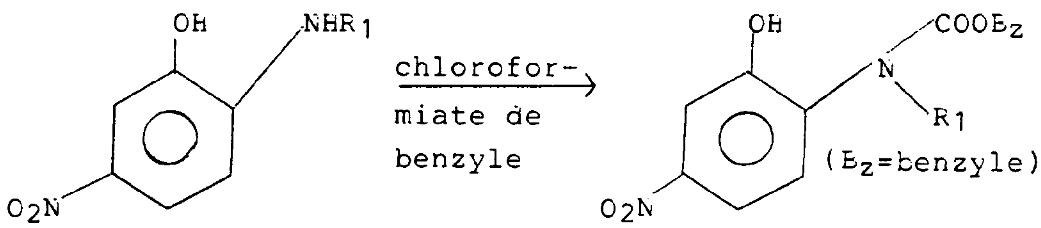


(V) (IV)

dans un solvant polaire, à une température ne dépassant pas 100°C, en présence d'un capteur d'acide chlorhydrique;
- en une deuxième étape, à réduire le nitrophénol de formule (IV) en aminophénol correspondant à une température de 20 à 100°C et obtenir le composé de formule (III); et
- en une troisième étape, acyler le composé de formule (III), selon la réaction :

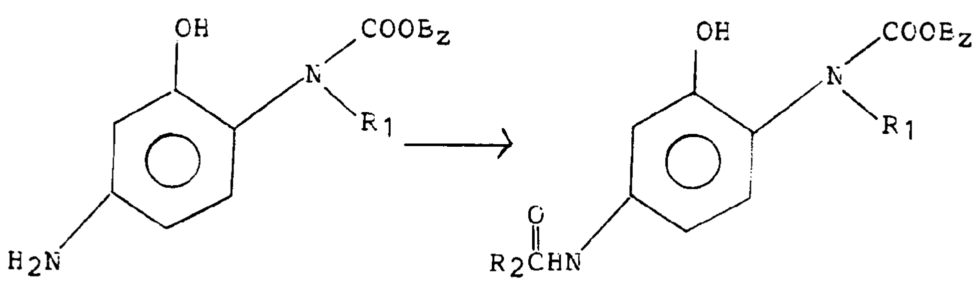

(III) (II)

- en une quatrième étape, remplacer le groupe $COOB_z$ (benzyloxycarbonyl) du composé de formule (II) par un hydrogène et obtenir le composé de formule (I).

**Revendications pour l'Etat contractant suivant : ES**

1. Procédé de préparation d'ortho-aminophénols 5-substitués, de formule :

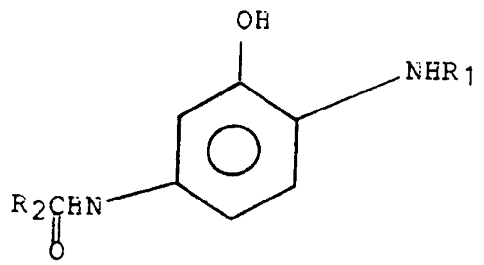

(I)

dans laquelle :

$R_1$ représente un atome d'hydrogène, un radical alkyle ayant 1 à 4 atomes de carbone, un radical hydroxyalkyle ayant 1 à 4 atomes de carbone;

$R_2$ représente, indépendamment de $R_1$, un radical alkyle ayant 1 à 4 atomes de carbone, un radical alcoxy ayant 1 à 4 atomes de carbone, un radical benzyle; consistant :

- en une première étape, à condenser du chloroformiate de benzyle sur l'aminonitrophénol de formule (V), selon la réaction :

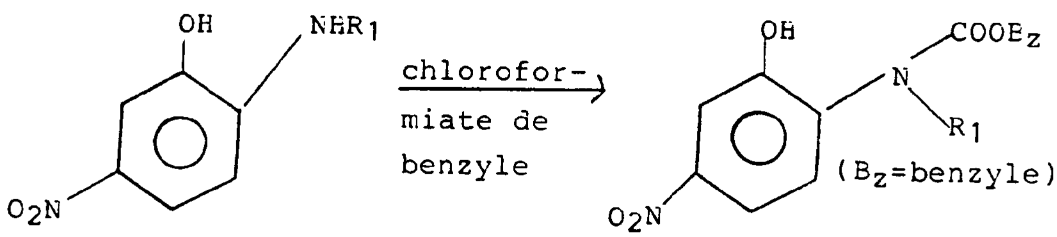


(V) (IV)

dans un solvant polaire, à une température ne dépassant pas 100°C, en présence d'un capteur d'acide chlorhydrique;

- en une deuxième étape, à réduire le nitrophénol de formule (IV) en aminophénol correspondant à une température de 20 à 100°C et obtenir le composé de formule (III); et
- en une troisième étape, acyler le composé de formule (III), selon la réaction :

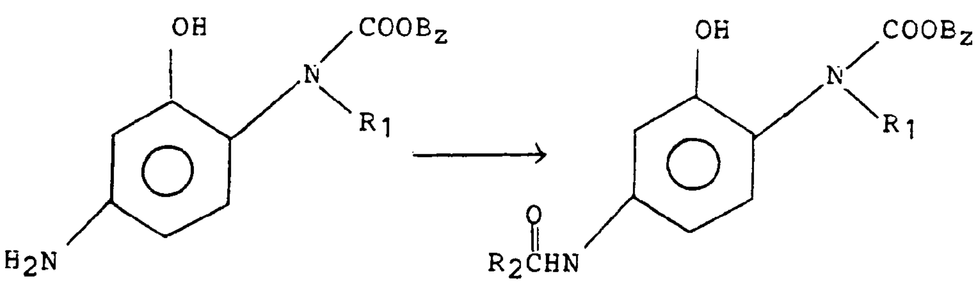

(III) (II)

- en une quatrième étape, remplacer le groupe $COOB_z$ (benzyloxycarbonyl) du composé de formule (II) par un hydrogène et obtenir le composé de formule (I); et si l'on désire en une cinquième étape, faire réagir l'ortho-aminophénol 5-substitué de formule (I) obtenu sur un acide ou sur un phénol pour produire le sel d'addition avec un acide ou le phénate correspondant.

2. Procédé de préparation selon la revendication 1 caractérisé en ce que l'on prépare un composé choisi parmi le 5-acétamido 2-aminophénol, le 2-amino 5-(éthoxycarbonyl)aminophénol, le 2-amino 5-(benzylcarbonyl)aminophénol, le 5-acétamido 2-(N-β-hydroxyéthyl)aminophénol, le 5-acétamido 2-(méthyl)aminophénol, leurs sels et leurs phénates.

3. Composition tinctoriale pour fibres kératiniques, en particulier pour cheveux humains, caractérisée par le fait qu'elle contient au moins un ortho-aminophénol 5-substitué, de formule :

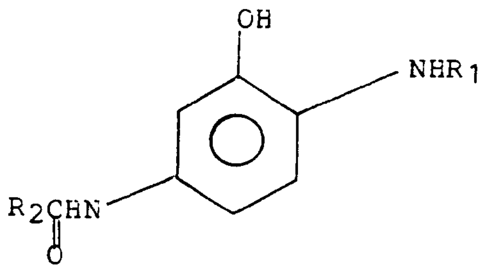

(I)

dans laquelle :

$R_1$ représente un atome d'hydrogène, un radical alkyle ayant 1 à 4 atomes de carbone, un radical hydroxyalkyle ayant 1 à 4 atomes de carbone;

$R_2$ représente, indépendamment de $R_1$, un radical alkyle ayant 1 à 4 atomes de carbone, un radical alcoxy ayant 1 à 4 atomes de carbone, un radical benzyle;

ou un sel d'addition avec un acide ou un phénate d'un composé de formule (I).

4. Composition tinctoriale selon la revendication 3, renfermant également un précurseur de colorant d'oxydation.

5. Composition tinctoriale selon la revendication 4, dans laquelle les précurseurs de colorants d'oxydation sont choisis parmi les paraphénylènediamines, les para-aminophénols, les orthophénylènediamines, les ortho-aminophénols, les bases hétérocycliques comme la 2,5-diaminopyridine.

6. Composition tinctoriale selon les revendications 3 à 5, renfermant également un ou plusieurs coupleurs.

7. Composition tinctoriale selon la revendication 6, dans laquelle les coupleurs sont choisis parmi les phénols, les métadiphénols, les méta-aminophénols, les métaphénylènediamines, les méta-acylaminophénols, les méta-uréidophénols, les métacarbalcoxyaminophénols, l' α-naphtol et les coupleurs possédant un groupe méthylène actif tel que les composés β-cétoniques et les pyrazolones; le 3,4-méthylènedioxyphénol, la3,4-méthylènedioxyaniline, le 2-bromo 4,5-méthylènedioxyphénol, le 2-chloro 4,5-méthylènedioxyphénol, la 2-méthoxy 4,5-méthylènedioxy aniline.

8. Composition tinctoriale selon les revendications 3 à 7, dans laquelle les ortho-aminophénols 5-substitués de formule (I) sont utilisés dans des concentrations comprises entre 0,02 et 6% et de préférence entre 0,15 et 5% en poids par rapport au poids total de la composition.

9. Composition tinctoriale selon les revendications 3 à 8, dans laquelle le poids total des précurseurs de colorants d'oxydation et des coupleurs est de 0,1 à 7% en poids par rapport au poids total de la composition.

10. Composition tinctoriale selon les revendications 3 à 9, dont le pH est compris entre 8 et 11 et de préférence entre 9 et 11.

11. Composition tinctoriale selon les revendications 3 à 10, renfermant également des adjuvants choisis dans le groupe formé par les agents tensio-actifs anioniques, cationiques, non-ioniques, amphotères et leurs mélanges, les solvants organiques, les agents épaississants, les agents antioxydants, les séquestrants, les tampons et les parfums.

12. Composition tinctoriale selon les revendications 3 à 11, se présentant sous forme de liquide, de crème, de gel ou bien est conditionnée en flacon aérosol en présence d'un agent propulseur.

13. Procédé de teinture des cheveux, caractérisé par le fait qu'il consiste, après avoir mélangé la composition tinctoriale telle que définie dans les revendications 3 à 12, avec l'agent oxydant choisi parmi l'eau oxygénée, le peroxyde d'urée et des persels, à appliquer le mélange résultant sur les cheveux pendant une

durée de pose de 10 à 40 minutes, à une température ne dépassant pas 40°C, après quoi on rince les cheveux, on les lave au shampooing, on les rince à nouveau et on les sèche.

**14.** Procédé de teinture des cheveux, caractérisé par le fait que l'on applique sur les cheveux, dans un premier temps, une composition tinctoriale selon les revendications 3, 8 et 10 à 12, contenant comme précurseur de colorant d'oxydation, uniquement des composés de formule (I) ou également d'autres précurseurs de colorants d'oxydation, et dans un second temps, on applique le ou les coupleur(s), l'agent oxydant étant présent dans la composition appliquée dans le second temps ou bien est appliqué sur les cheveux dans un troisième temps, chacune des compositions appliquées en premier et en second temps et, s'il y a lieu, l'agent oxydant appliqué en un troisième temps, sont laissés sur les cheveux pendant 10 à 40 minutes, à une température ne dépassant pas 40°C, après quoi on rince les cheveux, on les lave au shampooing, on les rince à nouveau et on les sèche.

## Patentansprüche

### Patentansprüche für folgende Vertragstaaten : AT, BE, CH, DE, FR, GB, GR, IT, LI, NL, SE

**1.** 5-Substituierte o-Aminophenole der Formel:

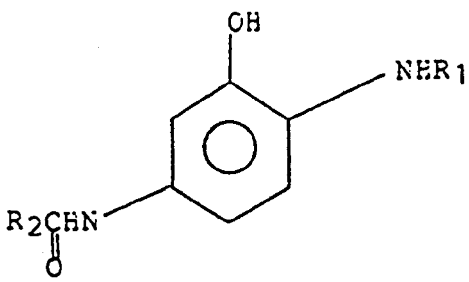

(I)

worin:

$R_1$ ein Wasserstoffatom, einen Alkylrest mit 1 bis 4 Kohlenstoffatomen, Hydroxyalkyrest mit 1 bis 4 Kohlenstoffatomen und

$R_2$, unabhängig von $R_1$, einen Alkyrest mit 1 bis 4 Kohlenstoffatomen, Alkoxyrest mit 1 bis 4 Kohlenstoffatomen oder Benzylrest darstellen,

deren Säureadditionssalze und Phenolate.

**2.** 5-Substituierte o-Aminophenole, gemäß Anspruch 1, ausgewählt aus 5-Acetamido-2-aminophenol, 2-Amino-5-(ethoxycarbonyl)aminophenol, 2-Amino-5-(benzylcarbonyl)aminophenol, 5-Acetamido-2-(N-β-hydroxyethylaminophenol, 5-Acetamido-2-(methyl)aminophenol und deren Salzen.

**3.** Färbezusammensetzung für keratinische Fasern, insbesondere für menschliche Haare, daurch **gekennzeichnet**, daß sie mindestens ein 5-substituiertes o-Aminophenol der Formel:

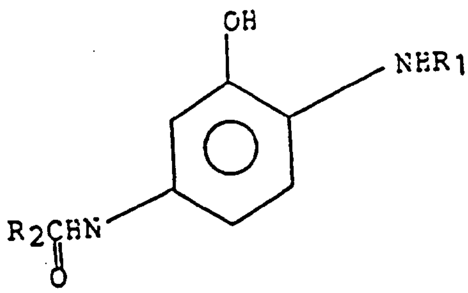

(I)

worin:

$R_1$ ein Wasserstoffatom, einen Alkylrest mit 1 bis 4 Kohlenstoffatomen, Hydroxyalkyrest mit 1 bis 4 Kohlenstoffatomen und

$R_2$, unabhängig von $R_1$, einen Alkyrest mit 1 bis 4 Kohlenstoffatomen, Alkoxyrest mit 1 bis 4 Kohlenstoffatomen oder Benzylrest darstellen, oder ein Additionssalz mit einer Säure oder ein Phenolat einer Verbindung der Formel (I) enthält.

4. Färbezusammensetzung gemäß Anspruch 3, enthaltend auch eine Oxidationsfarbstoff-Vorstufe.

5. Färbezusammensetzung gemäß Anspruch 4, worin die Oxidationsfarbstoff-Vorstufen ausgewählt sind aus p-Phenylendiaminen, p-Aminophenolen, o-Phenylendiaminen, o-Aminophenolen, heterocyclischen Basen wie 2,5-Diaminopyridin.

6. Färbezusammensetzung gemäß der Ansprüche 3 bis 5, enthaltend auch einen oder mehrere Kuppler.

7. Färbezusammensetzung gemäß Anspruch 6, worin die Kuppler ausgewählt sind unter Phenolen, m-Diphenolen, m-Aminophenolen, m-Phenylendiaminen, m-Acylaminophenolen, m-Ureidophenolen, m-Carbalkoxyaminophenolen, α-Naphthol und den Kupplern mit einer aktiven Methylengruppe, wie den β-Ketoverbindungen und Pyrazolonen; 3,4-Methylendioxyphenol, 3,4-Methylendioxyanilin, 2-Brom-4,5-methylendioxyphenol, 2-Chlor-4,5-methylendioxyphenol, 2-Methoxy-4,5-methylendioxyanilin.

8. Färbezusammensetzung gemäß der Ansprüche 3 bis 7, worin die 5-substituierten o-Aminophenole der Formel (I) in Konzentrationen von 0,02 bis 6 Gew.%, vorzugsweise 0,15 bis 5 Gew.%, bezogen auf das Gesamtgewicht der Zusammensetzung, verwendet werden.

9. Färbezusammensetzung gemäß der Ansprüche 3 bis 8, worin das Gesamtgewicht der Oxidationsfarbstoff-Vorstufen und Kuppler 0,1 bis 7 Gew.%, bezogen auf das Gesamtgewicht der Zusammensetzung, beträgt.

10. Färbezusammensetzung gemäß der Ansprüche 3 bis 9, deren pH 8 bis 11, vorzugsweise 9 bis 11, beträgt.

11. Färbezusammensetzung gemäß der Ansprüche 3 bis 10, enthaltend auch Hilfsstoffe, ausgewählt aus der Gruppe aus anionischen, kationischen, nicht-ionischen, amphoteren oberflächenaktiven Mitteln und deren Mischungen, organischen Lösungsmitteln, Verdickungsmitteln, Antioxidantien, Sequestriermitteln, Tampons und Parfüms.

12. Färbezusammensetzung gemäße der Ansprüche 3 bis 11, welche in Form einer Flüssigkeit, Creme, eines Gels vorliegt oder auch in einem Aerosolfläschchen in Gegenwart eines Treibmittelgases zubereitet ist.

13. Färbeverfahren der Haare, dadurch **gekennzeichnet**, daß es darauf beruht, daß man, nach Vermischung der in den Ansprüchen 3 bis 12 definierten Färbezusammensetzung mit einem Oxidationsmittel, ausgewählt aus mit Sauerstoff beladenem Wasser, Harnstoffperoxid und Persalzen, die entstandene Mischung auf die Haare während einer Verweilzeit von 10 bis 40 min bei einer Temperatur nicht über 40°C aufbringt, worauf man die Haare spült, sie unter Schamponieren wäscht, erneut spült und trocknet.

14. Färbeverfahren der Haare, dadurch **gekennzeichnet**, daß man auf die Haare, in einer ersten Stufe, eine Färbezusammensetzung gemäß der Ansprüche 3, 8 und 10 bis 12, enthaltend als Oxidationsfarbstoff-Vorstufe eine Verbindung der Formel (I) oder auch weitere Oxidationsfarbstoff-Vorstufen, und in einer zweiten Stufe den oder die Kuppler aufbringt, wobei das Oxidationsmittel in der in der zweiten Stufe aufgebrachten Zusammensetzung vorhanden ist oder auch auf die Haare in einer dritten Stufe aufgetragen wird, wobei die in der ersten und zweiten Stufe aufgebrachten Zusammensetzungen und, wenn dies stattfindet, das in der dritten Stufe aufgebrachte Oxidationsmittel auf den Haaren 10 bis 40 Minuten lang bei einer Temperatur nicht über 40°C gelassen werden, worauf man die Haare spült, sie unter Schamponieren wäscht, erneut spült und trocknet.

15. Verfahren zur Herstellung von 5-substituierten o-Aminophenolen der Formel:

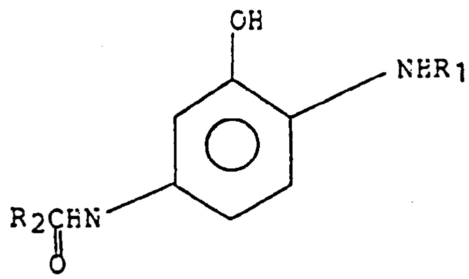

(I)

worin:

$R_1$ ein Wasserstoffatom, einen Alkylrest mit 1 bis 4 Kohlenstoffatomen, Hydroxyalkyrest mit 1 bis 4 Kohlenstoffatomen und

$R_2$, unabhängig von $R_1$, einen Alkyrest mit 1 bis 4 Kohlenstoffatomen, Alkoxyrest mit 1 bis 4 Kohlenstoffatomen oder Benzylrest darstellen, wobei man:

- in einer ersten Stufe Benzylchlorformiat mit einem Aminonitrophenol der Formel (V) gemäß der Reaktion:

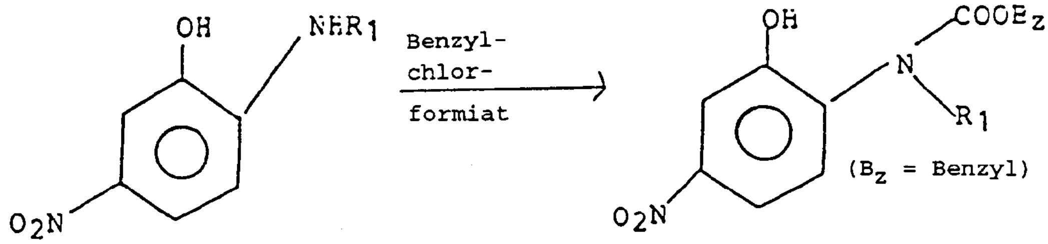


(V) (IV)

  in einem polaren Lösungsmittel bei einer Temperatur nicht über 100°C in Gegenwart eines Abfangmittels für Salzsäure kondensiert,
- in einer zweiten Stufe das Nitrophenol der Formel (V) zum entsprechenden Aminophenol bei einer Temperatur von 20 bis 100°C reduziert und die Verbindung der Formel (III) erhält,
- in einer dritten Stufe die Verbindung der Formel (III) gemäß der Reaktion acyliert:

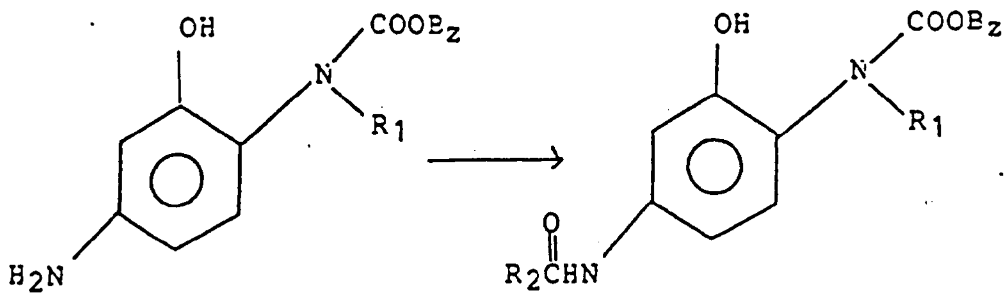

(III) (II)

- in einer vierten Stufe die $-COOB_z$-Gruppe (Benzyloxycarbonylgruppe) der Verbindung der Formel (II) durch ein Wasserstoffatom ersetzt und die Verbindung der Formel (I) erhält.

**Patentansprüche für folgenden Vertragsstaat : ES**

1. Verfahren zur Herstellung von 5-substituierten o-Aminophenolen der Formel:

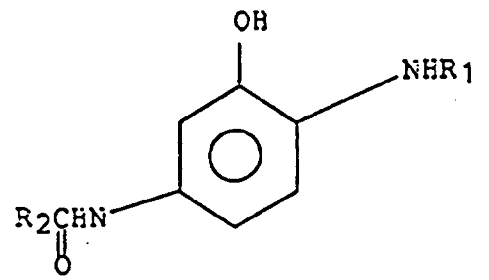

(I)

worin:

$R_1$ ein Wasserstoffatom, einen Alkylrest mit 1 bis 4 Kohlenstoffatomen, Hydroxyalkyrest mit 1 bis 4 Kohlenstoffatomen und

$R_2$, unabhängig von $R_1$, einen Alkyrest mit 1 bis 4 Kohlenstoffatomen, Alkoxyrest mit 1 bis 4 Kohlenstoffatomen oder Benzylrest darstellen, wobei man:

- in einer ersten Stufe Benzylchlorformiat mit einem Aminonitrophenol der Formel (V) gemäß der Reaktion:

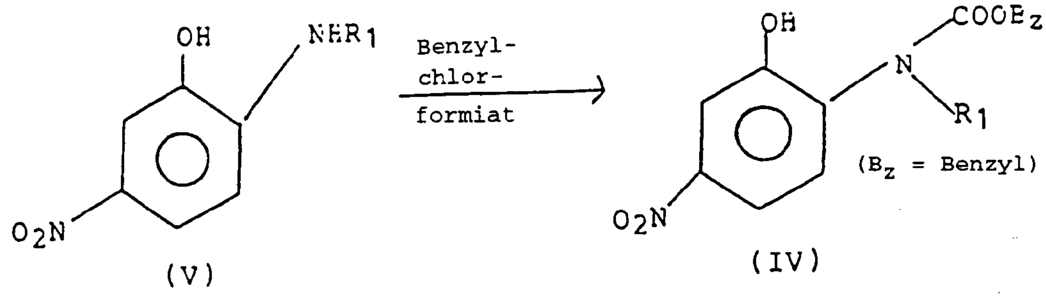


(V) (IV)

  in einem polaren Lösungsmittel bei einer Temperatur nicht über 100°C in Gegenwart eines Abfangmittels für Salzsäure kondensiert,
- in einer zweiten Stufe das Nitrophenol der Formel (V) zum entsprechenden Aminophenol bei einer Temperatur von 20 bis 100°C reduziert und die Verbindung der Formel (III) erhält,
- in einer dritten Stufe die Verbindung der Formel (III) gemäß der Reaktion acyliert:

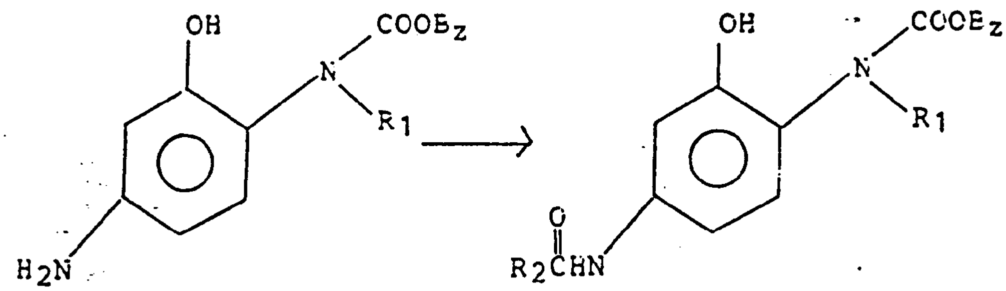

(III) (II)

- in einer vierten Stufe die $-COOB_z$-Gruppe (Benzyloxycarbonylgruppe) der Verbindung der Formel (II) durch ein Wasserstoffatom ersetzt und die Verbindung der Formel (I) erhält;

  und, falls gewünscht, in einer fünften Stufe, das erhaltene 5-substituierte o-Aminophenol der Formel (I) mit einer Säure oder einem Phenol reagieren läßt, um das Additionssalz mit einer Säure oder das entsprechende Phenolat herzustellen.

2. Herstellungsverfahren gemäß Anspruch 1, dadurch gekennzeichnet, daß man eine Verbindung herstellt, ausgewählt aus 5-Acetamido-2-aminophenol, 2-Amino-5-(ethoxycarbonyl)aminophenol, 2-Amino-5-(benzylcarbonyl)aminophenol, 5-Acetamido-2-(N-ß-hydroxyethylaminophenol, 5-Acetamido-2-(methyl)aminophenol, deren Salzen und Phenolaten.

3. Färbezusammensetzung für keratinische Fasern, insbesondere für menschliche Haare, dadurch **gekennzeichnet**, daß sie im mindestens ein 5-substituiertes o-Aminophenol der Formel:

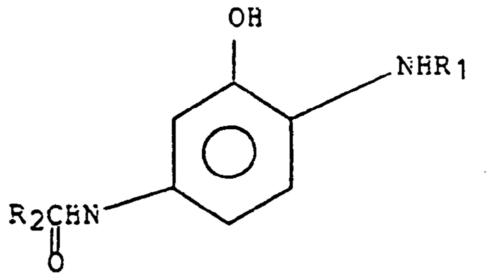

(I)

worin:

$R_1$ ein Wasserstoffatom, einen Alkylrest mit 1 bis 4 Kohlenstoffatomen, Hydroxyalkyrest mit 1 bis 4 Kohlenstoffatomen und

$R_2$, unabhängig von $R_1$, einen Alkyrest mit 1 bis 4 Kohlenstoffatomen, Alkoxyrest mit 1 bis 4 Kohlenstoffatomen oder Benzylrest darstellen, oder ein Additionssalz mit einer Säure oder ein Phenolat einer Verbindung der Formel (I) enthält.

4. Färbezusammensetzung gemäß Anspruch 3, enthaltend auch eine Oxidationsfarbstoff-Vorstufe.

5. Färbezusammensetzung gemäß Anspruch 4, worin die Oxidationsfarbstoff-Vorstufen ausgewählt sind aus p-Phenylendiaminen, p-Aminophenolen, o-Phenylendiaminen, o-Aminophenolen, heterocyclischen Basen wie 2,5-Diaminopyridin.

6. Färbezusammensetzung gemäß der Ansprüche 3 bis 5, enthaltend auch einen oder mehrere Kuppler.

7. Färbezusammensetzung gemäß Anspruch 6, worin die Kuppler ausgewählt sind unter Phenolen, m-Diphenolen, m-Aminophenolen, m-Phenylendiaminen, m-Acylaminophenolen, m-Ureidophenolen, m-Carboxyaminophenolen, α-Naphthol und den Kupplern mit einer aktiven Methylengruppe, wie den β-Ketoverbindungen und Pyrazolonen; 3,4-Methylendioxyphenol, 3,4-Methylendioxyanilin, 2-Brom-4,5-methylendioxyphenol, 2-Chlor-4,5-methylendioxyphenol, 2-Methoxy-4,5-methylendioxyanilin.

8. Färbezusammensetzung gemäß der Ansprüche 3 bis 7, worin die 5-substituierten o-Aminophenole der Formel (I) in Konzentrationen von 0,02 bis 6 Gew.%, vorzugsweise 0,15 bis 5 Gew.%, bezogen auf das Gesamtgewicht der Zusammensetzung, verwendet werden.

9. Färbezusammensetzung gemäß der Ansprüche 3 bis 8, worin das Gesamtgewicht der Oxidationsfarbstoff-Vorstufen und Kuppler 0,7 bis 7 Gew.%, bezogen auf das Gesamtgewicht der Zusammensetzung, beträgt.

10. Färbezusammensetzung gemäß der Ansprüche 3 bis 9, deren pH 8 bis 11, vorzugsweise 9 bis 11, beträgt.

11. Färbezusammensetzung gemäß der Ansprüche 3 bis 10, enthaltend auch Hilfsstoffe, ausgewählt aus der Gruppe aus anionischen, kationischen, nicht-ionischen, amphoteren oberflächenaktiven Mitteln und deren Mischungen, organischen Lösungsmitteln, Verdickungsmitteln, Antioxidantien, Sequestriermitteln, Tampons und Parfüms.

12. Färbezusammensetzung gemäße der Ansprüche 3 bis 11, welche in Form einer Flüssigkeit, Creme, eines Gels vorliegt oder auch in einem Aerosolfläschchen in Gegenwart eines Treibmittelgases zubereitet ist.

13. Färbeverfahren der Haare, dadurch **gekennzeichnet**, daß es darauf beruht, daß man, nach Vermischung der in den Ansprüchen 3 bis 12 definierten Färbezusammensetzung mit einem Oxidationsmittel, ausgewählt aus mit Sauerstoff beladenem Wasser, Harnstoffperoxid und Persalzen, die entstandene Mischung auf die Haare während einer Verweilzeit von 10 bis 40 min bei einer Temperatur nicht über 40°C aufbringt, worauf man die Haare spült, sie unter Schamponieren wäscht, erneut spült und trocknet.

**14.** Färbeverfahren der Haare, dadurch **gekennzeichnet**, daß man auf die Haare, in einer ersten Stufe, eine Färbezusammensetzung gemäß der Ansprüche 3, 8 und 10 bis 12, enthaltend als Oxidationsfarbstoff-Vorstufe eine Verbindung der Formel (I) oder auch weitere Oxidationsfarbstoff-Vorstufen und in einer zweiten Stufe den oder die Kuppler aufbringt, wobei das Oxidationsmittel in der in der zweiten Stufe aufgebrachten Zusammensetzung vorhanden ist oder auch auf die Haare in einer dritten Stufe aufgetragen wird, wobei die in der ersten und zweiten Stufe aufgebrachten Zusammensetzungen und, wenn dies stattfindet, das in der dritten Stufe aufgebrachte Oxidationsmittel auf den Haaren 10 bis 40 Minuten lang bei einer Temperatur nicht über 40°C gelassen werden, worauf man die Haare spült, sie unter Schamponieren wäscht, erneut spült und trocknet.

**Claims**

**Claims for the following Contracting States : AT, BE, CH, DE, FR, GB, GR, IT, LI, NL, SE**

**1.** 5-Substituted ortho-aminophenols of formula:

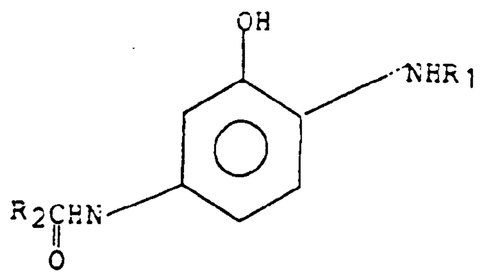

(I)

in which:

$R_1$ denotes a hydrogen atom, an alkyl radical having 1 to 4 carbon atoms or a hydroxyalkyl radical having 1 to 4 carbon atoms; and

$R_2$ denotes, independently of $R_1$, an alkyl radical having 1 to 4 carbon atoms, an alkoxy radical having 1 to 4 carbon atoms or a benzyl radical;

their addition salts with an acid and their phenates.

**2.** 5-Substituted ortho-aminophenols according to Claim 1, selected from 5-acetamido-2-aminophenol, 2-amino-5-(ethoxycarbonylamino)phenol, 2-amino-5-(benzylcarbonylamino)phenol, 5-acetamido-2-[N-(β-hydroxyethyl)amino]phenol, 5-acetamido-2-(methylamino)phenol and their salts.

**3.** Dyeing composition for keratinous fibres, especially for human hair, characterized in that it contains at least one 5-substituted ortho-aminophenol of formula:

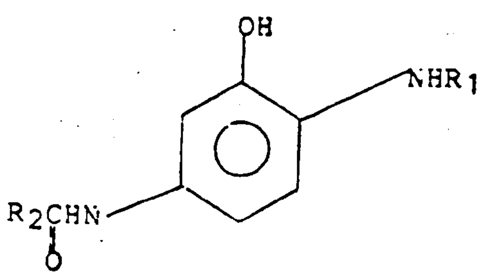

(I)

in which:

$R_1$ denotes a hydrogen atom, an alkyl radical having 1 to 4 carbon atoms or a hydroxyalkyl radical having 1 to 4 carbon atoms; and

$R_2$ denotes, independently of $R_1$, an alkyl radical having 1 to 4 carbon atoms, an alkoxy radical having 1 to 4 carbon atoms or a benzyl radical;

or an addition salt with an acid or a phenate of a compound of formula (I).

4. Dyeing composition according to Claim 3, also containing an oxidation dye precursor.

5. Dyeing composition according to Claim 4, in which the oxidation dye precursors are selected from para-phenylenediamines, para-aminophenols, ortho-phenylenediamines, ortho-aminophenols and heterocyclic bases such as 2,5-diaminopyridine.

6. Dyeing composition according to Claims 3 to 5, also containing one or more couplers.

7. Dyeing composition according to Claim 6, in which the couplers are selected from phenols, meta-diphenols, meta-aminophenols, meta-phenylenediamines, metaacylaminophenols, meta-ureidophenols, meta-carbalkoxyaminophenols, α-naphthol and couplers possessing an active methylene group such as β-keto compounds and pyrazolones; 3,4-methylenedioxyphenol, 3,4-methylenedioxyaniline,2-bromo-4,5-methylenedioxyphenol,2-chloro-4,5-methylenedioxyphenol and 2-methoxy-4,5-methylenedioxyaniline.

8. Dyeing composition according to Claims 3 to 7, in which the 5-substituted ortho-aminophenols of formula (I) are used in concentrations of between 0.02 and 6%, and preferably between 0.15 and 5%, by weight relative to the total weight of the composition.

9. Dyeing composition according to Claims 3 to 8, in which the total weight of the oxidation dye precursors and the couplers is from 0.1 to 7% by weight relative to the total weight of the composition.

10. Dyeing composition according to Claims 3 to 9, the pH of which is between 8 and 11, and preferably between 9 and 11.

11. Dyeing composition according to Claims 3 to 10, also containing adjuvants selected from the group composed of anionic, cationic, nonionic and amphoteric surfactants and mixtures thereof, organic solvents, thickeners, antioxidants, sequestering agents, buffers and fragrances.

12. Dyeing composition according to Claims 3 to 11, presented in the form of a liquid, cream or gel or alternatively packaged in an aerosol can in the presence of a propellant.

13. Hair dyeing process, characterized in that it consists, after the dyeing composition as defined in Claims 3 to 12 has been mixed with the oxidizing agent selected from hydrogen peroxide, urea peroxide and persalts, in applying the resulting mixture on the hair for an exposure period of 10 to 40 minutes, at a temperature not exceeding 40°C, after which the hair is rinsed, washed with shampoo, rinsed again and dried.

14. Hair dyeing process, characterized in that a dyeing composition according to Claims 3, 8 and 10 to 12, containing as an oxidation dye precursor only compounds of formula (I) or other oxidation dye precursors also, is applied on the hair in a first step, and the coupler or couplers is/are applied in a second step, the oxidizing agent being present in the composition applied in the second step or alternatively applied on the hair in a third step; each of the compositions applied in the first and second steps and, if appropriate, the oxidizing agent applied in a third step are left on the hair for 10 to 40 minutes, at a temperature not exceeding 40°C, after which the hair is rinsed, washed with shampoo, rinsed again and dried.

15. Process for preparing 5-substituted ortho-aminophenols of formula:
according to Claim 1,

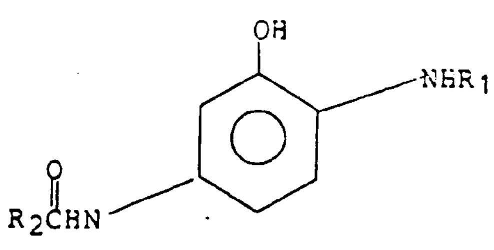

(I)

where $R_1$ denotes a hydrogen atom, an alkyl radical having from 1 to 4 carbon atoms or a hydroxyalkyl radical having from 1 to 4 carbon atoms; and

$R_2$ denotes, independently of $R_1$, an alkyl radical having from 1 to 4 carbon atoms, an alkoxy radical having from 1 to 4 carbon atoms or a benzyl radical,
which consists:

- in a first stage, in condensing benzyl chloroformate with the aminonitrophenol of formula (V), according to the reaction:

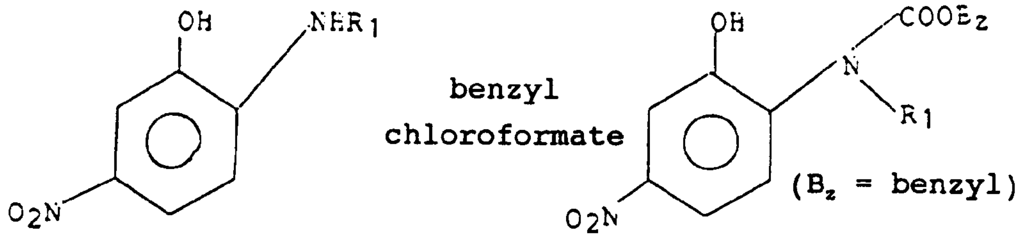


(V) (IV)

in a polar solvent, at a temperature not exceeding 100°C, in the presence of a trapping agent for hydrochloric acid;

- in a second stage, in reducing the nitrophenol of formula (IV) to the corresponding aminophenol, at a temperature of 20 to 100°C, and obtaining the compound of formula (III); and
- in a third stage, in acylating the compound of formula (III), according to the reaction:

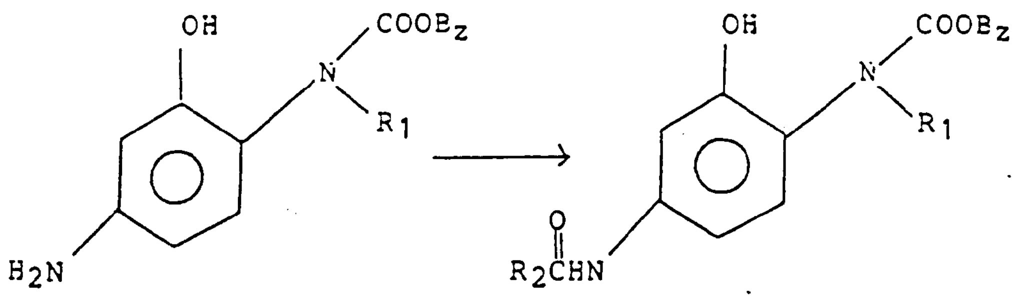

(III) (II)

- in a fourth stage, in replacing the $COOB_z$ (benzyloxycarbonyl) group in the compound of formula (II) by a hydrogen and obtaining the compound of formula (I).

**Claims for the following Contracting State : ES**

**1.** Process for preparing 5-substituted orthoaminophenols of formula:

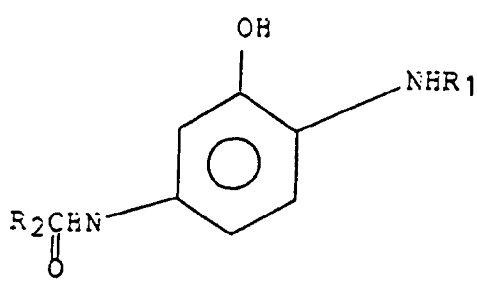

(I)

in which:

$R_1$ denotes a hydrogen atom, an alkyl radical having from 1 to 4 carbon atoms or a hydroxyalkyl radical having from 1 to 4 carbon atoms; and

$R_2$ denotes, independently of $R_1$, an alkyl radical having from 1 to 4 carbon atoms, an alkoxy radical having from 1 to 4 carbon atoms or a benzyl radical; which consists:

- in a first stage, in condensing benzyl chloroformate with the aminonitrophenol of formula (V), accord-

ing to the reaction:

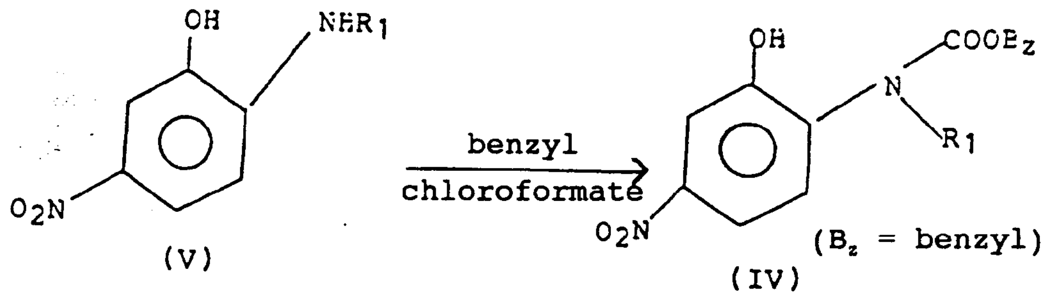


(V) (IV) ($B_z$ = benzyl)

in a polar solvent, at a temperature not exceeding 100°C, in the presence of a trapping agent for hydrochloric acid;

- in a second stage, in reducing the nitrophenol of formula (IV) to the corresponding aminophenol, at a temperature of 20 to 100°C, and obtaining the compound of formula (III); and
- in a third stage, in acylating the compound of formula (III), according to the reaction:

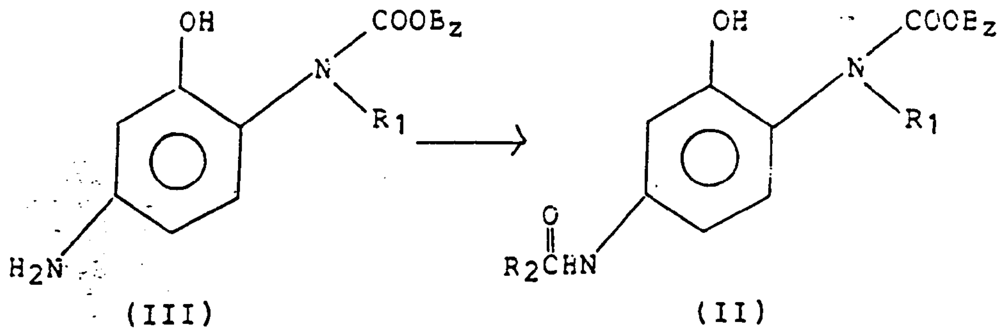

(III) (II)

- in a fourth stage, in replacing the $COOB_z$ (benzyloxycarbonyl) group in the compound of formula (II) by a hydrogen and obtaining the compound of formula (I); and, if desired, in a fifth stage, in reacting the 5-substituted ortho-aminophenol of formula (I) obtained, with an acid or with a phenol to produce the addition salt with an acid or the corresponding phenate.

**2.** Preparation process according to Claim 1, characterized in that a compound is prepared which is selected from 5-acetamido-2-aminophenol, 2-amino-5-(ethoxycarbonylamino)phenol, 2-amino-5-(benzylcarbonylamino)phenol, 5-acetamido-2-[N-(β-hydroxyethyl)amino]phenol, 5-acetamido-2-(methylamino)phenol, their salts and their phenates.

**3.** Dyeing composition for keratinous fibres, especially for human hair, characterized in that it contains at least one 5-substituted ortho-aminophenol of formula:

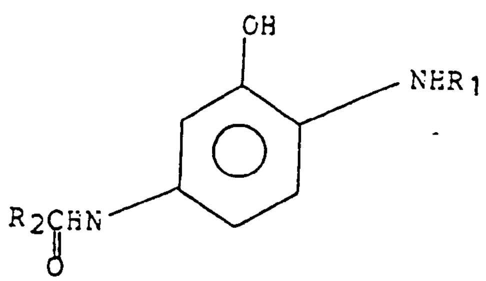

(I)

in which:

$R_1$ denotes a hydrogen atom, an alkyl radical having 1 to 4 carbon atoms or a hydroxyalkyl radical having 1 to 4 carbon atoms; and

$R_2$ denotes, independently of $R_1$, an alkyl radical having 1 to 4 carbon atoms, an alkoxy radical having 1 to 4 carbon atoms or a benzyl radical;
or an addition salt with an acid or a phenate of a compound of formula (I).

4. Dyeing composition according to Claim 3, also containing an oxidation dye precursor.

5. Dyeing composition according to Claim 4, in which the oxidation dye precursors are selected from para-phenylenediamines, para-aminophenols, ortho-phenylenediamines, ortho-aminophenols and heterocyclic bases such as 2,5-diaminopyridine.

6. Dyeing composition according to Claims 3 to 5, also containing one or more couplers.

7. Dyeing composition according to Claim 6, in which the couplers are selected from phenols, meta-diphenols, meta-aminophenols, meta-phenylenediamines, metaacylaminophenols, meta-ureidophenols, meta-carbalkoxyaminophenols, α-naphthol and couplers possessing an active methylene group such as β-keto compounds and pyrazolones; 3,4-methylenedioxyphenol, 3,4-methylenedioxyaniline,2-bromo-4,5-methylenedioxyphenol,2-chloro-4,5-methylenedioxyphenol and 2-methoxy-4,5-methylenedioxyaniline.

8. Dyeing composition according to Claims 3 to 7, in which the 5-substituted ortho-aminophenols of formula (I) are used in concentrations of between 0.02 and 6%, and preferably between 0.15 and 5%, by weight relative to the total weight of the composition.

9. Dyeing composition according to Claims 3 to 8, in which the total weight of the oxidation dye precursors and the couplers is from 0.1 to 7% by weight relative to the total weight of the composition.

10. Dyeing composition according to Claims 3 to 9, the pH of which is between 8 and 11, and preferably between 9 and 11.

11. Dyeing composition according to Claims 3 to 10, also containing adjuvants selected from the group composed of anionic, cationic, nonionic and amphoteric surfactants and mixtures thereof, organic solvents, thickeners, antioxidants, sequestering agents, buffers and fragrances.

12. Dyeing composition according to Claims 3 to 11, presented in the form of a liquid, cream or gel or alternatively packaged in an aerosol can in the presence of a propellant.

13. Hair dyeing process, characterized in that it consists, after the dyeing composition as defined in Claims 3 to 12 has been mixed with the oxidizing agent selected from hydrogen peroxide, urea peroxide and persalts, in applying the resulting mixture on the hair for an exposure period of 10 to 40 minutes, at a temperature not exceeding 40°C, after which the hair is rinsed, washed with shampoo, rinsed again and dried.

14. Hair dyeing process, characterized in that a dyeing composition according to Claims 3, 8 and 10 to 12, containing as an oxidation dye precursor only compounds of formula (I) or other oxidation dye precursors also, is applied on the hair in a first step, and the coupler or couplers is/are applied in a second step, the oxidizing agent being present in the composition applied in the second step or alternatively applied on the hair in a third step; each of the compositions applied in the first and second steps and, if appropriate, the oxidizing agent applied in a third step are left on the hair for 10 to 40 minutes, at a temperature not exceeding 40°C, after which the hair is rinsed, washed with shampoo, rinsed again and dried.